# EUROPEAN PATENT SPECIFICATION

(11) **EP 4 399 198 B1**
(45) Date of publication and mention of the grant of the patent: **04.11.2026**
(21) Application number: 22865863.9
(22) Date of filing: 06.09.2022
(51) Int. Cl.: C07C 39/04, C07C 259/06, C07C 259/04, A61P 35/00, A61K 31/166, A61K 31/47, C07C 259/10, C07D 317/66

(54) **DUAL INHIBITOR OF HISTONE DEACETYLASE 6 AND HEAT SHOCK PROTEIN 90**
DUALER INHIBITOR DER HISTONDEACETYLASE 6 UND HITZESCHOCKPROTEIN 90
INHIBITEUR DOUBLE DE L'HISTONE DÉSACÉTYLASE 6 ET DE LA PROTÉINE DE CHOC THERMIQUE 90

(30) Priority: 06.09.2021 US 202163241066 P
(43) Date of publication of application: 17.07.2024
(73) Proprietor: Taipei Medical University, Taipei City 110 (TW)
(72) Inventor: CHUANG, Kuo-Hsiang, Taipei City, 110 (TW); LIOU, Jing-Ping, Taipei City, 110 (TW); YEN, Yun, Arcadia, CA 91006 (US)
(74) Representative: Lavoix
(86) International application number: PCT/US2022/075962
(87) International publication number: WO 2023/035005

(56) References cited:
- WO-A1-2021/013163
- US-A1- 2021 009 539
- MEHNDIRATTA SAMIR ET AL: "N-alkyl-hydroxybenzoyl anilide hydroxamates as dual inhibitors of HDAC and HSP90, downregulating IFN-[gamma] induced PD-L1 expression", EUROPEAN JOURNAL OF MEDICINAL CHEMISTRY, ELSEVIER MASSON, AMSTERDAM, NL, vol. 185, 24 September 2019 (2019-09-24), XP085935523, ISSN: 0223-5234, [retrieved on 20190924], DOI: 10.1016/J.EJMECH.2019.111725
- OJHA RITU ET AL: "Isoindoline scaffold-based dual inhibitors of HDAC6 and HSP90 suppressing the growth of lung cancer in vitro and in vivo", EUROPEAN JOURNAL OF MEDICINAL CHEMISTRY, ELSEVIER MASSON, AMSTERDAM, NL, vol. 190, 23 January 2020 (2020-01-23), XP086065709, ISSN: 0223-5234, [retrieved on 20200123], DOI: 10.1016/J.EJMECH.2020.112086
- MEHNDIRATTA SAMIR; LIN MEI-HSIANG; WU YI-WEN; CHEN CHUN-HAN; WU TUNG-YUN; CHUANG KUO-HSIANG; CHAO MIN-WU; CHEN YI-YING; PAN SHIOW-: "N-alkyl-hydroxybenzoyl anilide hydroxamates as dual inhibitors of HDAC and HSP90, downregulating IFN-γ induced PD-L1 expression", EUROPEAN JOURNAL OF MEDICINAL CHEMISTRY, vol. 185, 24 September 2019 (2019-09-24), AMSTERDAM, NL , pages 1 - 14, XP085935523, ISSN: 0223-5234, DOI: 10.1016/j.ejmech.2019.111725
- OJHA RITU; NEPALI KUNAL; CHEN CHUN-HAN; CHUANG KUO-HSIANG; WU TUNG-YUN; LIN TONY EIGHT; HSU KAI-CHENG; CHAO MIN-WU; LAI MEI-JUNG; : "Isoindoline scaffold-based dual inhibitors of HDAC6 and HSP90 suppressing the growth of lung cancer in vitro and in vivo", EUROPEAN JOURNAL OF MEDICINAL CHEMISTRY, vol. 190, 23 January 2020 (2020-01-23), AMSTERDAM, NL , pages 1 - 19, XP086065709, ISSN: 0223-5234, DOI: 10.1016/j.ejmech.2020.112086
- WU TUNG-YUN, CHEN MICHAEL, CHEN I-CHUNG, CHEN YI-JOU, CHEN CHE-YI, WANG CHANG-HUNG, CHENG JING-JY, NEPALI KUNAL, CHUANG KUO-HSIANG: "Rational design of synthetically tractable HDAC6/HSP90 dual inhibitors to destroy immune-suppressive tumor microenvironment", JOURNAL OF ADVANCED RESEARCH, vol. 46, 1 April 2023 (2023-04-01), AMSTERDAM, NL , pages 159 - 171, XP093063341, ISSN: 2090-1232, DOI: 10.1016/j.jare.2022.06.009

## Description

### PRIORITY

This application claims the benefit of priority to U.S. Provisional Application No. 63/241,066, filed on 6 September 2021.

### Field of the Invention

The present disclosure relates generally to a pharmaceutical compound. Particularly, the present disclosure relates to a dual inhibitor of histone deacetylase 6 and heat shock protein 90.

### Background of the Invention

Tumorigenesis is a complex and dynamic process, consisting of three stages: initiation, progression, and metastasis. Tumors are encircled by extracellular matrix (ECM) and stromal cells, and the physiological state of the tumor microenvironment (TME) is closely connected to every step of tumorigenesis. The TME is the ecosystem that surrounds a tumor inside the body. It includes immune cells, the extracellular matrix, blood vessels and other cells, like fibroblasts. Due to the severity of the condition, development of an effective strategy to manage TME and thus treat cancer is critical. N-alkyl-hydroxybenzoyl anilide hydroxamates are known as dual inhibitors of HDAC and HSP90, downregulating IFN-γ induced PD-L1 expression (Mehndiratta, et al, European Journal of Medicinal Chemistry 185 (2020) 111725)*.*

### Summary of the Invention

The present disclosure provides a dual inhibitor for removing immune suppression from tumor microenvironments or stimulating an immune system against tumors.

In one aspect, the present disclosure recites a compound of formula (I), 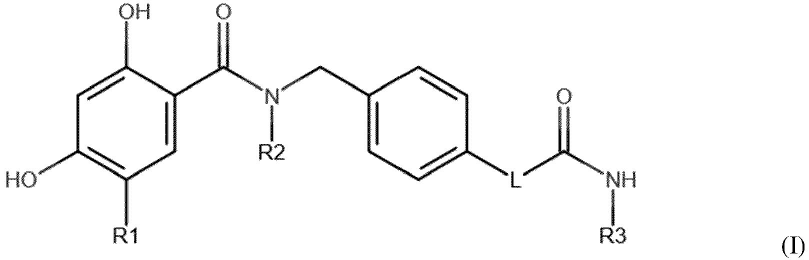
or pharmaceutically acceptable salts, solvates, hydrates, polymorphs, tautomers, stereoisomers, or isotopically enriched derivatives,
wherein:
   R₁ is hydrogen or alkyl,
   R₂ is 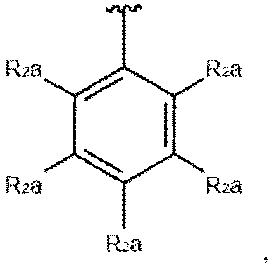 wherein R₂ₐ each independently represents hydrogen, halogen, hydroxy, alkyl, alkoxy, NR'R", heterocyclyl or aryl, or two adjacent R₂ₐ, together with the carbon atoms of the phenyl to which they attached, form a heterocyclyl fused to the phenyl, wherein the heterocyclyl and aryl are unsubstituted or substituted with alkyl, halogen, hydroxy, NR'R", alkoxy or hydroxy, wherein the heterocyclyl contains at least one heteroatom selected from the group consisting of N, O, S and any combination thereof, wherein R' and R" independently represent hydrogen or alkyl;
   L is 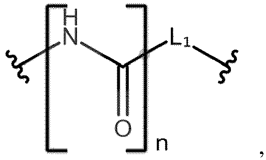 wherein n is 0 or 1, L₁ is a bond, alkylene or alkenylene, with the proviso that when n is 1, L₁ is not a bond, and
   R₃ is OH or phenyl optionally substituted with halogen, hydroxy, alkyl, alkoxy, or NR'R", wherein R' and R" independently represent hydrogen or alkyl.

In some embodiments of the disclosure, R₁ is methyl, ethyl, n-propyl, isopropyl, n-butyl, 2-butyl or 2,2-dimethylethyl.

In some embodiments of the disclosure, R₂ is C₁₋₆ alkyl or

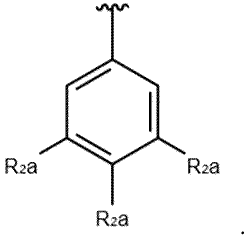

In some embodiments of the disclosure, R₂ is phenyl, flourophenyl, chlorophenyl, iodophenyl, benzodioxol, morpholinophenyl, (dimethylamino)phenyl, piperidinylphenyl, methoxyphenyl, or hydroxyphenyl.

In some embodiments of the disclosure, L is a bond, or L is 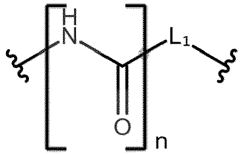 where n is 1 and L₁ is C₁-C₆ alkylene or C₂-C₆ alkenylene.

In some embodiments of the disclosure, R₃ is OH or a phenyl substituted with NR'R".

In some embodiments of the disclosure, R₁ is C₁-C₆ alkyl, R₂ is 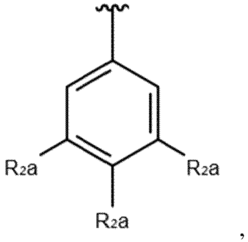 L is a bond, and R₃ is phenyl optionally substituted with halogen, hydroxy, alkyl, alkoxy, or NR'R", wherein R' and R" independently represent hydrogen or alkyl.

In some embodiments of the disclosure, R₁ is C₁-C₆ alkyl, R₂ is 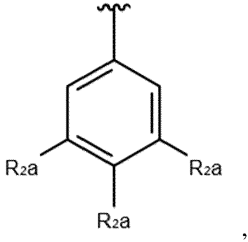 L is a bond or alkenylene and R₃ is OH.

In some embodiments of the disclosure, R₁ is C₁-C₆ alkyl, R₂ is 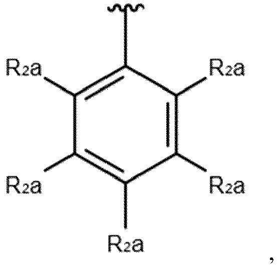 L is 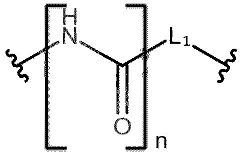 where n is 1 and L₁ is C₁-C₆ alkylene and R₃ is OH.

In some embodiments of the disclosure, R₁ is isopropyl, R₂ is 4-methoxyphenyl, L is 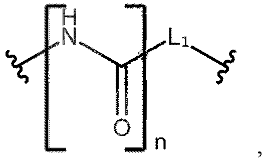 wherein (1) n is 1 and L₁ is alkylene, or (2) n is 0 and L₁ is alkenylene, and R₃ is OH.

Examples of the compound include, but are not limited to, the following: 4-dihydroxy-N-(4-(hydroxycarbamoyl)benzyl)-5-isopropyl-N-methylbenzamide, N-ethyl-2,4-dihydroxy-N-(4-(hydroxycarbamoyl)benzyl)-5-isopropylbenzamide, 2,4-dihydroxy-N-(4-(hydroxycarbamoyl)benzyl)-5-isopropyl-N-propylbenzamide, 2,4-dihydroxy-N-(4-(hydroxycarbamoyl)benzyl)-5-isopropyl-N-phenylbenzamide, N-(4-fluorophenyl)-2,4-dihydroxy-N-(4-(hydroxycarbamoyl)benzyl)-5-isopropylbenzamide, N-(4-chlorophenyl)-2,4-dihydroxy-N-(4-(hydroxycarbamoyl)benzyl)-5-isopropylbenzamide, 2,4-dihydroxy-N-(4-(hydroxycarbamoyl)benzyl)-N-(4-iodophenyl)-5-isopropylbenzamide, N-(benzo[d][1,3]dioxol-5-yl)-2,4-dihydroxy-N-(4-(hydroxycarbamoyl)benzyl)-5-isopropylbenzamide, 2,4-dihydroxy-N-(4-(hydroxycarbamoyl)benzyl)-5-isopropyl-N-(4-morpholinophenyl)benzamide, N-(4-(dimethylamino)phenyl)-2,4-dihydroxy-N-(4-(hydroxycarbamoyl)benzyl)-5-isopropylbenzamide, 2,4-dihydroxy-N-(4-(hydroxycarbamoyl)benzyl)-5-isopropyl-N-(4-(4-methylpiperazin-1-yl)phenyl)benzamide, 2,4-dihydroxy-N-(4-(hydroxycarbamoyl)benzyl)-5-isopropyl-N-(4-(piperidin-1-yl)phenyl)benzamide, 2,4-dihydroxy-N-(4-hydroxycarbamoyl-benzyl)-5-isopropyl-N-(4-methoxy-phenyl)-benzamide, and 2,4-dihydroxy-N-(4-hydroxycarbamoyl-benzyl)-N-(4-hydroxy-phenyl)-5-isopropyl-benzamide.

The present disclosure also provides a pharmaceutical composition comprising a therapeutically effective amount of the compound of formula (I) as a dual inhibitor and optionally pharmaceutically acceptable excipients.

While not claimed, the present disclosure also provides a method for amelioration and/or treatment of tumors in a subject in need of such amelioration and/or treatment, the method comprising administering the pharmaceutical composition as described herein to the subject. In some embodiments of the disclosure, the method further comprises administering an immune checkpoint inhibitor, particularly wherein the immune checkpoint is PD-1. In some embodiments of the disclosure, the method further comprises administering a tumor-targeting inhibitor. An example of the tumor-targeting inhibitor is an antibody such as an anti-EGFR antibody. In some embodiments of the disclosure, the method further comprises administering an anti-cancer drug such as chemotherapy drugs, hormone therapy drugs, immunotherapy drugs, and tumor-specific inhibitors. Examples of the chemotherapy drugs include, but are not limited to, an alkylating agent, antimetabolite, anthracycline, topoisomerase I and II inhibitor, mitotic inhibitor, platinum based drug, steroid or anti-angiogenic agent. Examples of the immunotherapy drugs include, but are not limited to, PD-1 inhibitors, PD-L1 inhibitors, CTLA-4 inhibitors and TF inhibitors. In another aspect, the present disclosure provides use of the pharmaceutical composition as described herein in the manufacture of a medicament for amelioration and/or treatment of tumors in a subject in need of such amelioration and/or treatment.

While not claimed, the present disclosure also provides a method for removing immune suppression from tumor microenvironments or stimulating an immune system against tumors in a subject in need of such removal or stimulation, the method comprising administering the pharmaceutical composition as described herein to the subject. In another aspect, the present disclosure provides use of the pharmaceutical composition as described herein in the manufacture of a medicament for removing immune suppression from tumor microenvironments or stimulating an immune system against tumors in a subject in need of such removal or stimulation.

While not claimed, the present disclosure also provides a method for inhibiting histone deacetylase 6 (HDAC6) in tumor microenvironments in a subject in need of such inhibition, the method comprising administering the pharmaceutical composition as described herein to the subject. Furthermore, in some embodiments of the disclosure, the method is for blocking signal transducer and activator of STAT1 pathway induced by IFN-γ. In some embodiments of the disclosure, the method is for lowering programmed death ligand 1 (PD-L1) or indoleamine-pyrrole 2,3-dioxygenase (IDO) expression of the tumor. In some embodiments of the disclosure, the method is for inhibiting acetylation of α-tubulin and histone. In another aspect, the present disclosure provides use of the pharmaceutical composition as described herein in the manufacture of a medicament for inhibiting HDAC6 in tumor microenvironments in a subject in need of such inhibition.

While not claimed, the present disclosure provides a method for inducing infiltration of cytotoxic T cells in tumor microenvironments in a subject in need of such induction, the method comprising administering the pharmaceutical composition as described herein to the subject. In some embodiments of the disclosure, the method is for inducing granzyme B expression. In another aspect, the present disclosure provides use of the pharmaceutical composition as described herein in the manufacture of a medicament for inducing infiltration of cytotoxic T cells in tumor microenvironments in a subject in need of such induction.

While not claimed, the present disclosure provides a method for inhibiting heat shock protein 90 (HSP90) in tumor microenvironments in a subject in need of such inhibition, the method comprising administering the pharmaceutical composition as described herein to the subject. In some embodiments of the disclosure, the method is for destabilizing proteins for tumor growth. In some embodiments of the disclosure, the method is for increasing heat shock protein 70 (HSP70) expression. Furthermore, in some embodiments of the disclosure, the method is for reducing expression of Src, AKT, retinoblastoma protein (Rb) or focal adhesion kinase (FAK). In another aspect, the present disclosure provides use of the pharmaceutical composition as described herein in the manufacture of a medicament for inhibiting HSP90 in tumor microenvironments in a subject in need of such inhibition.

While not claimed, the present disclosure provides a method for inhibiting tumor growth in a subject in need of such inhibition, the method comprising administering the pharmaceutical composition as described herein to the subject. In some embodiments of the disclosure, the tumor is a solid tumor. Examples of the tumor include, but are not limited to, colorectal cancer, pancreatic carcinoma, small cell lung cancer, non-small cell lung cancer, renal cell carcinoma, breast cancer, head and neck cancer, prostate cancer, malignant gliomas, osteosarcoma, gastric cancer, malignant mesothelioma, multiple myeloma, ovarian cancer, synovial sarcoma, thyroid cancer, or melanoma. In another aspect, the present disclosure provides use of the pharmaceutical composition as described herein in the manufacture of a medicament for inhibiting tumor growth in a subject in need of such inhibition.

While not claimed, the present disclosure provides a method for inhibiting tumor recurrence in a subject in need of such inhibition, the method comprising administering the pharmaceutical composition as described herein to the subject. In another aspect, the present disclosure provides use of the pharmaceutical composition as described herein in the manufacture of a medicament for inhibiting tumor recurrence in a subject in need of such inhibition.

While not claimed, the present disclosure also provides a method for reducing Treg cell level in a subject in need of such reduction, the method comprising administering the pharmaceutical composition as described herein to the subject. In another aspect, the present disclosure provides use of the pharmaceutical composition as described herein in the manufacture of a medicament for reducing Treg cell level in a subject in need of such reduction.

While not claimed, the present disclosure also provides a method for increasing central memory T cell level in a subject in need of such increase, the method comprising administering the pharmaceutical composition as described herein to the subject. In another aspect, the present disclosure provides use of the pharmaceutical composition as described herein in the manufacture of a medicament for increasing central memory T cell level in a subject in need of such increase.

### Brief Description of the Drawings

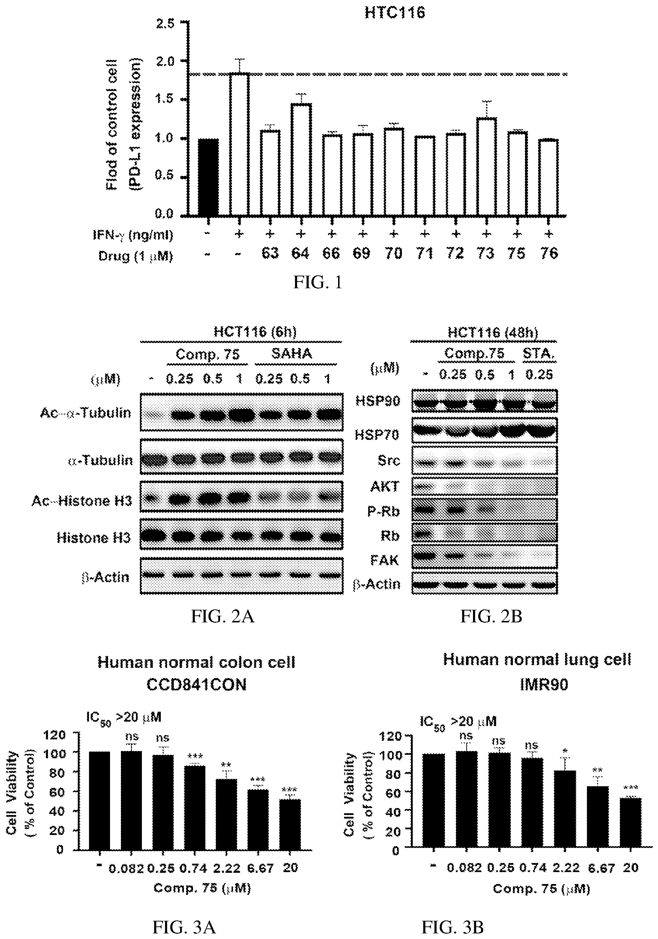

FIG. 1 shows that HSP90/HDAC dual inhibitors inhibit PD-L1 expression in IFN-γ-induced colorectal cancer cells. The PD-L1 expression of HCT116 cells was induced by 100 ng/ml IFN-γ in a 6-well plate with 32 x 10⁴ cells/well, and then 1µM HSP90/HDAC dual inhibitors were administered for 48 hours. The PD-L1 expression of HCT116 cells was measured by flow cytometry for each derivative, SD.
FIGs. 2A and 2B show that Compound 75 inhibits HDAC6 and HSP90 activities. FIG. 2A shows that Compound 75 (0.25-1µM), positive control SAHA (0.25-1µM) or DMSO (control) were tested in 6-well plates, 32 x 10⁴ cells/well, in human colorectal cancer cells HCT116 for 6 hours by western blot analysis. The ability of α-tubulin and histone H3 acetylation was measured. FIG. 2B shows that various HSP90 client proteins (HSP90, HSP70, Src, AKT, p-Rb, Rb, FAK) were assayed with Compound 75 (0.25-1µM), positive control STA9090 (0.25µM) or DMSO (control) for 48 hours.
FIGs. 3A and 3B show the effects of Compound 75 on cytotoxicity to normal cell lines. Cells were treated with DMSO or different concentrations of Compound 75 for 48 hours and cell viability was assessed by MTT assay. FIG. 3A shows CCD841CON (human-derived colon cells); FIG. 3B shows IMR90 (human-derived lung cells). Data are expressed as mean ± S.D. N values greater than 3. * P < 0.05; ** P < 0.01; *** P < 0.001, compared with the control group.

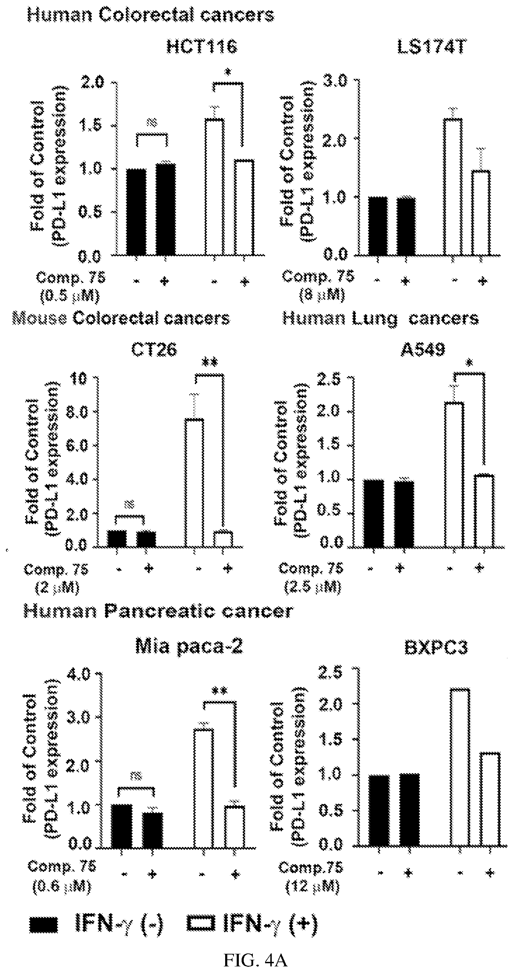

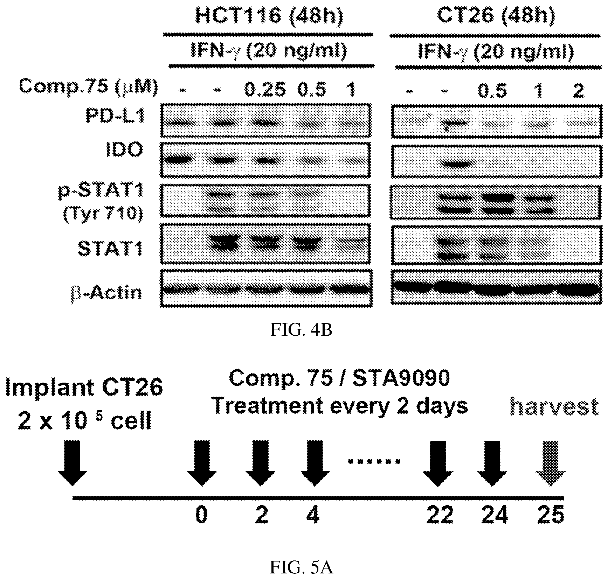

FIGs. 4A and 4B show that Compound 75 inhibits IFN-γ-induced PD-L1 and IDO expression. FIG. 4A shows that IFN-γ-induced PD-L1 expression in the cell membrane of human colon cancer cells (HCT116 and LS174T), human pancreatic cancer cells (Mia paca-2 and BXPC3), human lung cancer cells (A549) and mouse colon cancer cells (CT26 cells). The cells were tested in 6-well plates with 32 x 10⁴ cells/well and co-treated with Compound 75 for 48 hours and analyzed by flow cytometry. Bar, SD. FIG. 4B shows that PD-L1 and IDO expression of HCT116 and CT26 cells induced by IFN-γ in western blot analysis and co-treated with Compound 75 (at the indicated concentrations) for 48 hours.

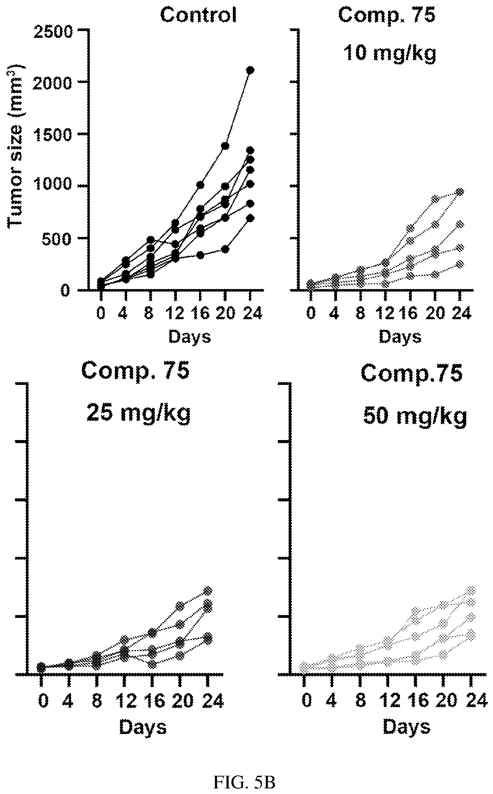

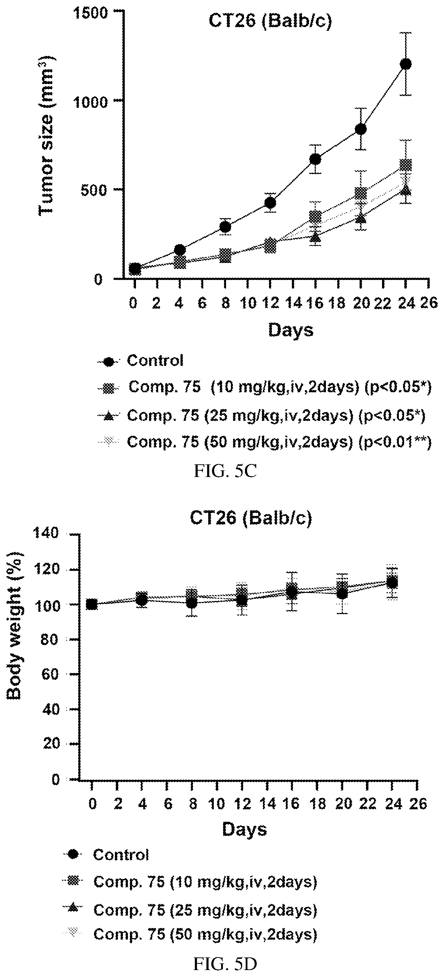

FIGs. 5A to 5D show that Compound 75 was used to inhibit the growth of colorectal cancer cells (CT26) tumors in immunocompetent mice (Balb/c). CT26 cells were injected subcutaneously into the back of mice at 2 x 10⁵ cells, and as shown in FIG. 5A, Compound 75 was administered at 10 mg/kg, 25 mg/kg, and 50 mg/kg every two days via intravenous tail injection to mice with colorectal cancer tumors (CT26, 50 mm³) for 24 days. Tumor size in each mouse (FIG. 5B) and mean tumor size (FIG. 5C) were measured periodically. Bar, SEM. FIG. 5D shows body weight of mice; Bar, SD. N values greater than 3.

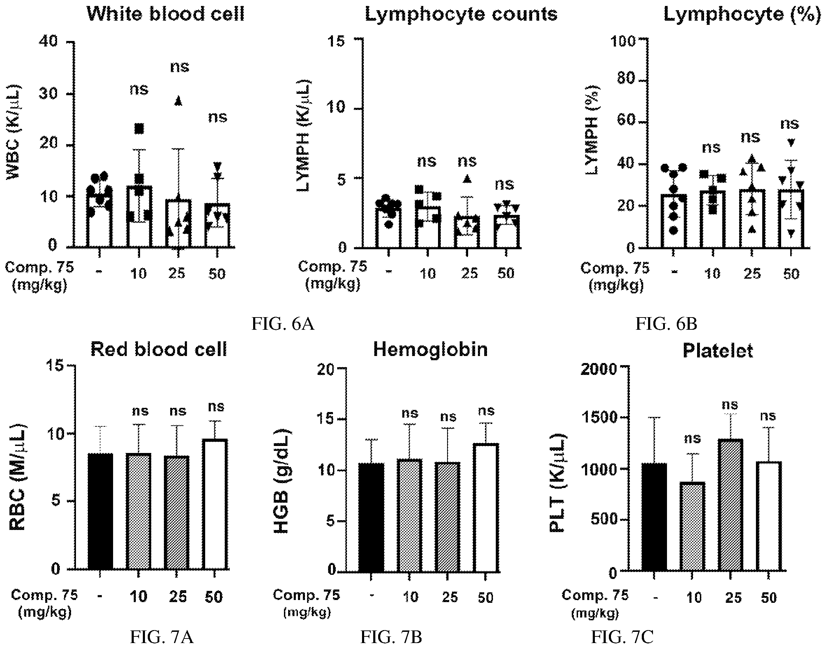

FIGs. 6A and 6B show the effects of Compound 75 on the white blood cell and lymphocyte counts. Compound 75 was administered to BALB/c mice with CT26 tumors every two days (10, 25, and 50 mg/kg), and blood was collected from the heart on day 25. Blood was analyzed for white blood cell and lymphocyte counts (as shown in FIG. 6A) and lymphocyte percentage (as shown in FIG. 6B).
FIGs. 7A to 7C show hematotoxicity analysis of Compound 75. Compound 75 was administered to BALB/c mice with CT26 tumors every two days (10, 25 and 50 mg/kg), and blood was collected from the heart on day 25 and analyzed for red blood cell (as shown in FIG. 7A), hemoglobin (FIG. 7B) and platelet (FIG. 7C) counts.

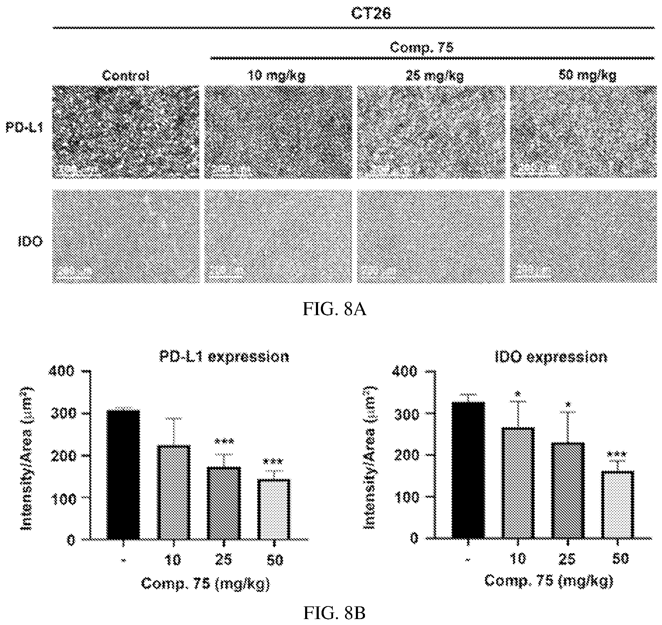

FIGs. 8A and 8B show that Compound 75 was administered intravenously via tail at 10 mg/kg, 25 mg/kg and 50 mg/kg every two days to mice with colorectal cancer tumors (CT26). The tumors were removed and immunohistochemically stained at 25 days after intravenous tail injection in mice with colorectal cancer tumors. FIG. 8A shows expression of PD-L1 and IDO (brown). FIG. 8B shows that quantitative analysis was performed using HistoQuest software. Bar, SD.

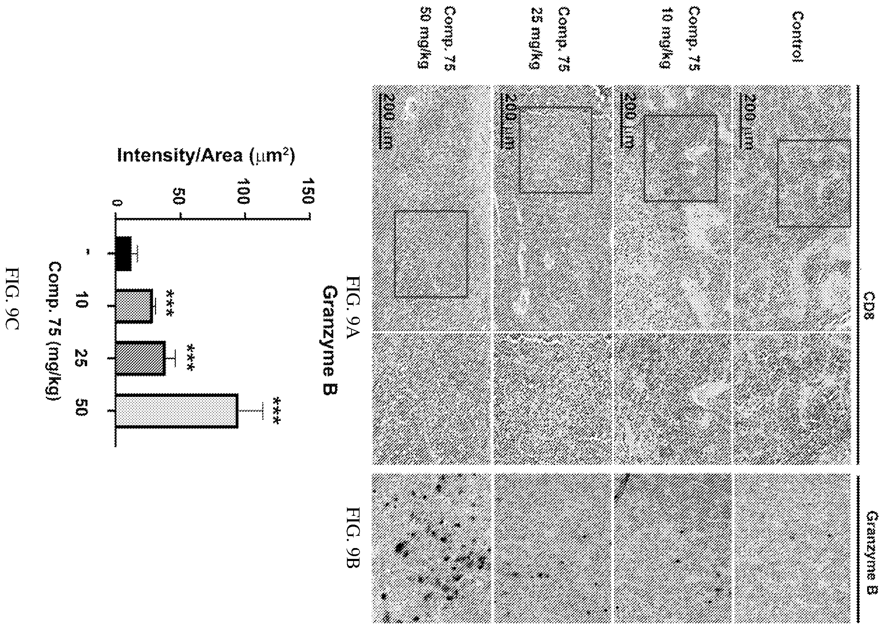

FIGs. 9A to 9C show that Compound 75 was administered intravenously via the tail once every two days at concentrations of 10 mg/kg, 25 mg/kg, and 50 mg/kg in immunocompetent mice (Balb/c) with colorectal cancer tumors (CT26). The tumors were removed from mice on day 25 and embedded in paraffin and sectioned for immunohistochemical staining. FIG. 9A shows the amount of CD8 cytotoxicity T cells. FIG. 9B shows expression of granzyme B. FIG. 9C shows that quantitative analysis was performed using HistoQuest software. Bar, SD.

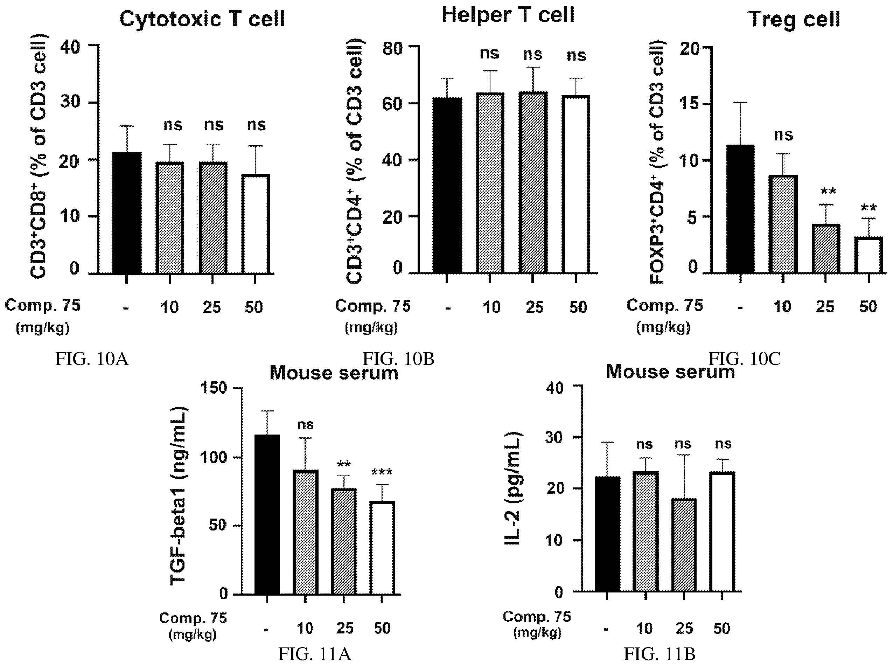

FIGs. 10A to 10C show immune cell regulation of peripheral blood by Compound 75. BALB/c mice with CT26 tumors were treated with 10, 25 and 50 mg/kg of Compound 75 every two days, and blood was collected from the heart and analyzed by flow cytometry on day 25 for cytotoxicity T cells (CD3⁺CD8⁺) (as shown in FIG. 10A), helper T cells (CD3⁺CD4⁺) (as shown in FIG. 10B) and regulatory T cells (CD3⁺CD4⁺FOXP3⁺) (as shown in FIG. 10C). Data are expressed as mean ± S.D. N values greater than 3. * P < 0.05; ** P < 0.01; *** P < 0.001, compared with the control group.
FIGs. 11A and 11B show effects of Compound 75 on peripheral blood cytokines. BALB/c mice with CT26 tumors were treated with 10, 25 and 50 mg/kg of Compound 75 every two days and sacrificed on day 25. Data are expressed as mean ± S.D. N values greater than 3. *P < 0.05; ** P < 0.01; *** P < 0.001, compared to the control group.

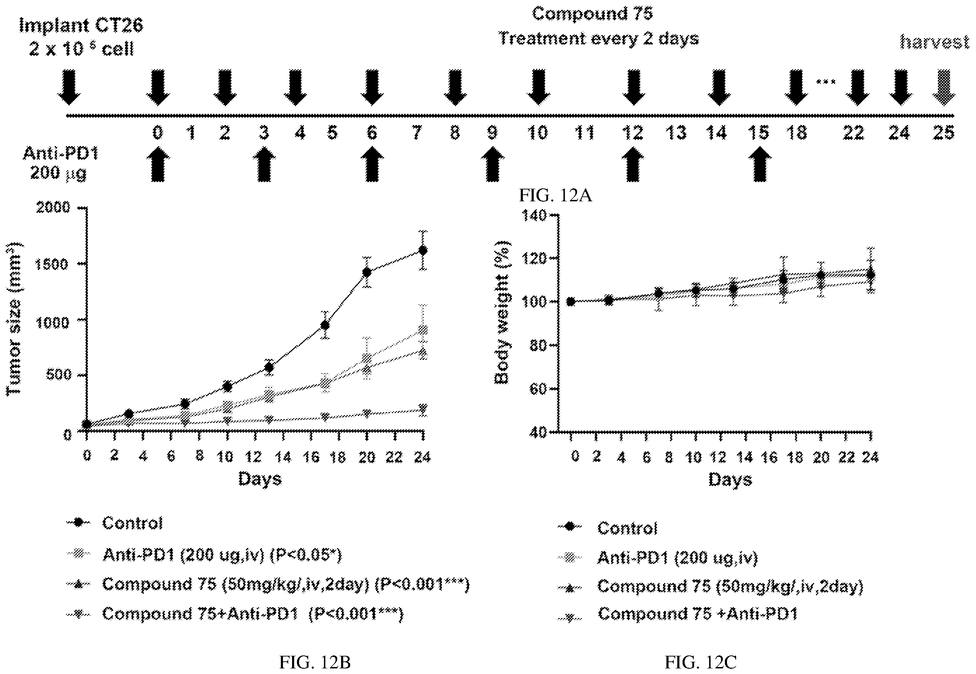

FIGs 12A to 12C show that Compound 75 enhances the effect of *in vivo* Anti-PD1 treatment. CT26 cells were injected subcutaneously with 2 x 10⁵ cells into the backs of immunocompetent mice (Balb/c), and Compound 75 was administered at 50 mg/kg every two days; Anti-PD1 200µg was administered every three days, and six doses of Anti-PD1 were administered intravenously through the tail to mice with colorectal cancer tumors (CT26, 50 mm³) for 24 days (as shown in FIG. 12A). Tumor size in each mouse (as shown in FIG. 12B) and mean tumor size (as shown in FIG. 12C) were measured periodically. Bar, SEM. (D) Body weight of mice; Bar, SD. N values greater than 3.

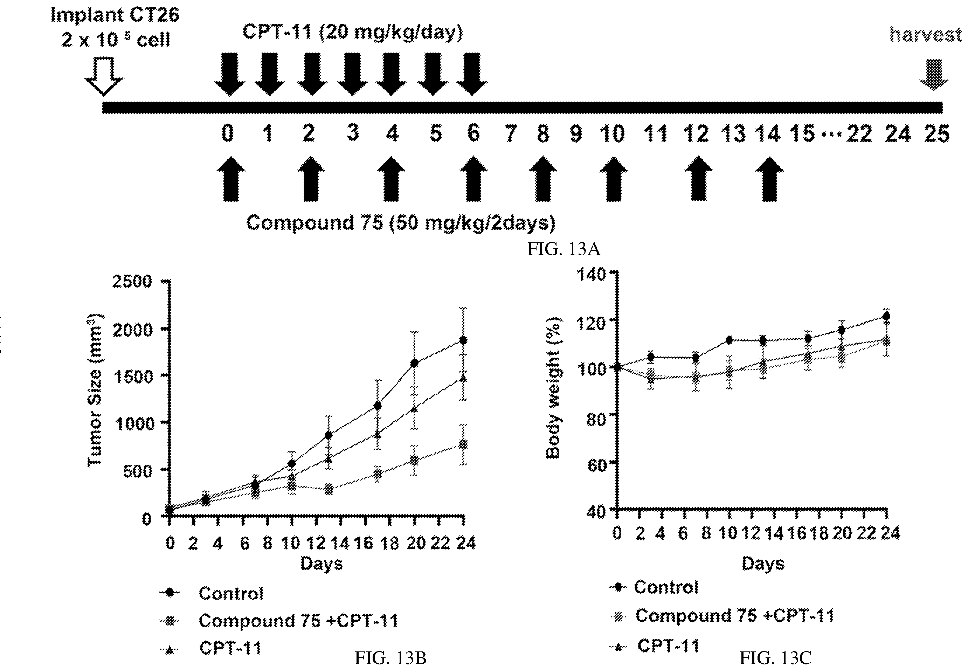

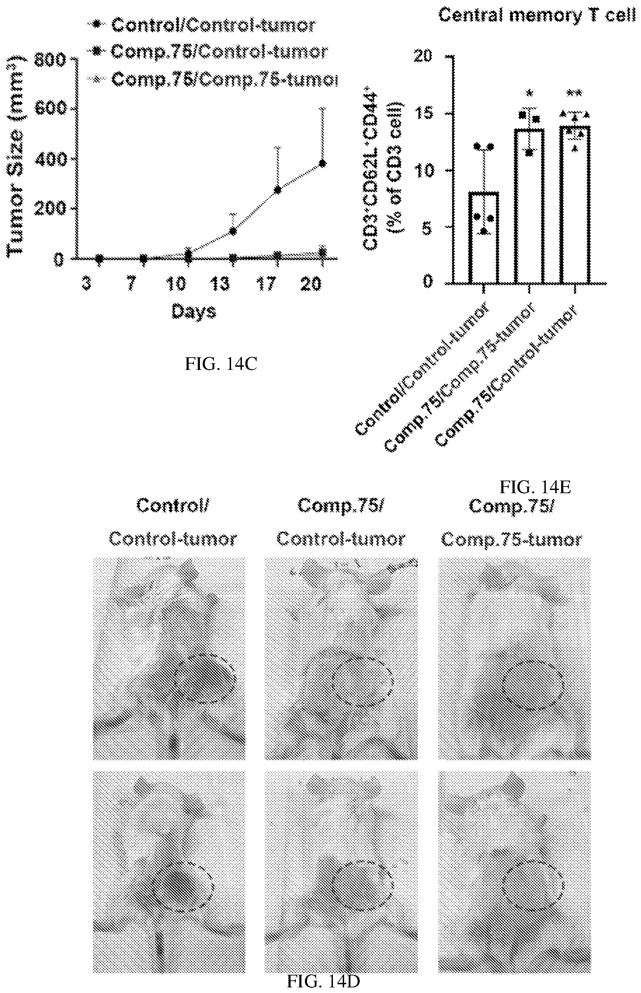

FIGs. 13A to 13C show that Compound 75 enhances the tumor suppression ability of chemotherapeutic agent CPT-11. CT26 was injected subcutaneously into immunocompetent mice (BALB/c). The mice were treated with CPT-11 or combined Compound 75 with CPT-11 when the tumor grew to 50-100 mm³. CPT-11 was given at 20 mg/kg for 7 consecutive days; compound 75 was given at 50 mg/kg every 2 days (8 doses in total, with suspension after day 14). The mice were sacrificed on day 25 (as shown in FIG. 13A). FIG. 13B shows mean tumor size. FIG. 14C shows body weight of mice. Bar, S.E.M.

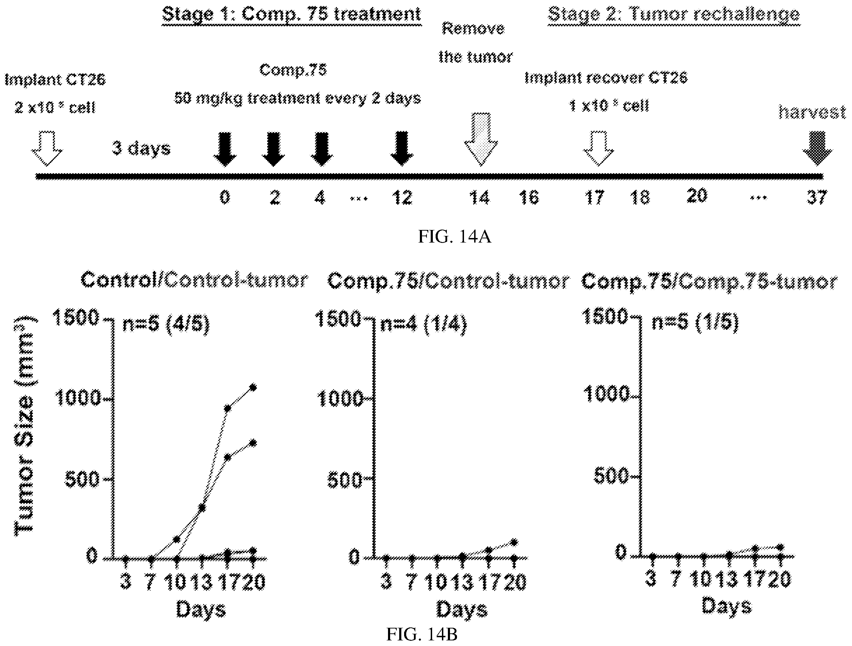

FIGs. 14A to 14E show that Compound 75 inhibits the recurrence of tumors. Stage 1: CT26 was injected subcutaneously into the left dorsum of immunocompetent mice (BALB/c), and Compound 75 treatment was started after 3 days, administered intravenously at a dose of 50 mg/kg every two days (7 doses in total). After tumor removal, tumor cells were isolated for *in vitro* culture (untreated tumor: control-tumor; Compound 75-treated tumor: Compound 75-tumor). Stage 2: On day 17, the isolated tumor cells were injected subcutaneously into the right side of the mouse back and the mice were sacrificed on day 37 (as shown in FIG. 14A). FIG. 14B shows volume of each tumor in each treatment group. FIG. 14C shows mean tumor size. FIG. 14D shows photographs of mouse backs on day 37. FIG. 14E shows the percentage of central memory T cells. Data are mean ± S.E.M. N values greater than 4.

### Detailed Description of the Invention

Unless defined otherwise, all scientific or technical terms used herein have the same meaning as those understood by persons of ordinary skill in the art to which the present invention belongs. Any method or material similar or equivalent to those described herein can be understood and used by those of ordinary skill in the art to practice the present invention.

It must be noted that, as used in the specification and the appended claims, the singular forms "a," "an" and "the" include plural referents unless the context clearly dictates otherwise. Thus, unless otherwise required by context, singular terms shall include the plural and plural terms shall include the singular.

As used herein, the term "optional" or "optionally" means that the subsequently described event or circumstance may or may not occur, and that the description includes instances where the said event or circumstance occurs and instances where it does not. For example, the phrase "optionally comprising an agent" means that the agent may or may not exist.

The term "and/or" is used to refer to both things or either one of the two mentioned.

Often, ranges are expressed herein as from "about" one particular value and/or to "about" another particular value. When such a range is expressed, an embodiment includes the range from the one particular value and/or to the other particular value. Similarly, when values are expressed as approximations, by use of the word "about," it will be understood that the particular value forms another embodiment. It will be further understood that the endpoints of each of the ranges are significant both in relation to and independently of the other endpoint. As used herein, the term "about" refers to ± 20%, preferably ± 10%, and even more preferably ± 5%.

The terms "treatment," "treating," and "treat" generally refer to obtaining a desired pharmacological and/or physiological effect. The effect may be preventive in terms of completely or partially preventing a disease, disorder, or symptom thereof, and may be therapeutic in terms of a partial or complete cure for a disease, disorder, and/or symptoms attributed thereto. "Treatment" used herein covers any treatment of a disease in a mammal, preferably a human, and includes (1) suppressing development of a disease, disorder, or symptom thereof in a subject or (2) relieving or ameliorating the disease, disorder, or symptom thereof in a subject.

The terms "individual," "subject," and "patient" herein are used interchangeably and refer to any mammalian subject for whom diagnosis, treatment, or therapy is desired.

As used herein, the term "in need of treatment" refers to a judgment made by a caregiver (e.g., physician, nurse, nurse practitioner, or individual in the case of humans; veterinarian in the case of animals, including non-human mammals), and such judgment is that a subject requires or will benefit from treatment. This judgment is made based on a variety of factors that are in the realm of a caregiver's expertise, but that include the knowledge that the subject is ill, or will be ill, as the result of a condition that is treatable by the compounds of the present disclosure.

The term "administering" includes routes of administration which allow the active ingredients of the disclosure to perform their intended function.

As used in the present invention, the term "pharmaceutical composition" refers to a mixture containing a therapeutic agent administered to an animal, for example a human, for treating or eliminating a particular disease or pathological condition that the animal suffers.

The term "effective amount" of an active ingredient as provided herein refers to a sufficient amount of the ingredient to provide the desired regulation of a desired function. As will be pointed out below, the exact amount required will vary from subject to subject, depending on the disease state, physical conditions, age, sex, species and weight of the subject, the specific identity and formulation of the composition, etc. Dosage regimens may be adjusted to induce the optimum therapeutic response. For example, several divided doses may be administered daily or the dose may be proportionally reduced as indicated by the exigencies of the therapeutic situation. Thus, it is not possible to specify an exact "effective amount." However, an appropriate effective amount can be determined by one of ordinary skill in the art using only routine experimentation.

The term "pharmaceutically acceptable" as used herein refers to compounds, materials, compositions, and/or dosage forms which are, within the scope of sound medical judgment, suitable for use in contact with the tissues of a subject (either a human or non-human animal) without excessive toxicity, irritation, allergic response, or other problems or complications, commensurate with a reasonable benefit/risk ratio. Each carrier, excipient, etc., must also be "acceptable" in the sense of being compatible with the other ingredients of the formulation. Suitable carriers, excipients, etc., can be found in standard pharmaceutical texts.

As used herein, the phrase "unsubstituted or substituted" means that substitution is optional. In the event a substitution is desired, then such substitution means that any number of hydrogens on the designated atom is replaced with a selection from the indicated group, provided that the normal valence of the designated atom is not exceeded, and that the substitution results in a stable compound.

Unless otherwise specified, the term "alkyl" as used herein refers to a monovalent, saturated, straight or branched chain hydrocarbon radical containing 1 to 12 carbon atoms. Preferably, the alkyl is a C₁-C₈ alkyl group. More preferably, the alkyl is a C₁-C₆ alkyl group. The alkyl can be substituted or unsubstituted. Examples of a C₁-C₆ alkyl group include, but are not limited to, methyl, ethyl, *n*-propyl, isopropyl, *n*-butyl, isobutyl, *sec-*butyl, *t*-butyl, pentyl (including all isomeric forms), and hexyl (including all isomeric forms), heptyl (including all isomeric forms), and octyl (including all isomeric forms).

As used herein, the terms "heterocyclic ring" and "heterocyclyl" are used interchangeably. The term "heterocyclic ring" or "heterocyclyl" refers to a mono-, bi-, or polycyclic structure having from 3 to 14 atoms, alternatively 3 to 12 atoms, alternatively 3 to 10 atoms, alternatively 3 to 8 atoms, alternatively 4 to 7 atoms, alternatively 5 or 6 atoms; wherein one or more atoms, for example 1, 2 or 3 atoms, are independently selected from the group consisting of N, O, and S, the remaining ring-constituting atoms being carbon atoms. The ring structure may be saturated or unsaturated, but is not aromatic. Exemplary heterocyclic rings include, but are not limited to, imidazolyl, imidazolinoyl, imidazolidinyl, quinolyl, isoqinolyl, indolyl, indazolyl, indazolinolyl, perhydropyridazyl, pyridazyl, pyridyl, pyrrolyl, pyrrolinyl, pyrrolidinyl, pyrazolyl, pyrazinyl, quinoxolyl, piperidinyl, pyranyl, pyrazolinyl, piperazinyl, pyrimidinyl, pyridazinyl, morpholinyl, thiamorpholinyl, furyl, thienyl, triazolyl, thiazolyl, carbolinyl, tetrazolyl, thiazolidinyl, benzofuranoyl, thiamorpholinyl sulfone, oxazolyl, benzoxazolyl, oxopiperidinyl, oxopyrrolidinyl, oxoazepinyl, azepinyl, isoxozolyl, isothiazolyl, furazanyl, tetrahydropyranyl, tetrahydrofuranyl, thiazolyl, thiadiazoyl, dioxolyl, dioxinyl, oxathiolyl, benzodioxolyl, dithiolyl, thiophenyl, tetrahydrothiophenyl, sulfolanyl, dioxanyl, dioxolanyl, tetahydrofurodihydrofuranyl, tetrahydropyranodihydrofuranyl, dihydropyranyl, tetradyrofurofuranyl, and tetrahydropyranofuranyl.

As used herein, the terms "halide" and "halo" are used interchangeably and include fluoro, chloro, bromo and iodo.

Unless otherwise specified, the term "alkoxy" as used herein refers to radicals of the general formula -O-(alkyl), wherein alkyl is as defined above. Exemplary alkoxy includes, but is not limited to, methoxy, ethoxy, n-propoxy, isopropoxy, n-butoxy, iso-butoxy, sec-butoxy, tert-butoxy, n-pentoxy, and n-hexoxy.

The TME, being full of immunosuppressive cells and protein, makes T-cell activation difficult and can even inhibit anti-cancer efforts. Therefore, TME has presented a dilemma in cancer therapy. When immune cells attack cancer cells, they secrete interferon gamma (IFN-γ) which induces the expression of Programmed death-ligand 1 (PD-L1) through the signal transducer and activator of transcription 1 (STAT1) pathway. Afterwards, PD-L1 binds to PD-1 (Programmed death 1) on the surface of T cells and further inhibits the anticancer effect of T cells (Juneja, et al. J Exp Med 214, 895-904, 2017)*.* It has also been clinically demonstrated that the higher the PD-L1 expression in the tumor area, the worse the prognosis of the patient (Shen et al., World J Surg Oncol 17, 4, 2019)*.* In another aspect, IFN-γ also induces indoleamine 2,3-dioxygenase (IDO) secretion by the tumor, which depletes tryptophan in the tumor microenvironment and promotes the production of kynurenine. It results in inhibition of T cell growth and induction of T cell apoptosis, leading to an immune escape mechanism (Prendergast, et al., Oncogene 26; 27(28):3889-900, 2008)*.*

Provided herein are dual inhibitors that inhibit histone deacetylase 6 and heat shock protein 90 for amelioration and/or treatment of tumors through removal of immune suppression from tumor microenvironments or stimulation of an immune system against tumors. In one aspect, the present disclosure provides a compound of formula (I), 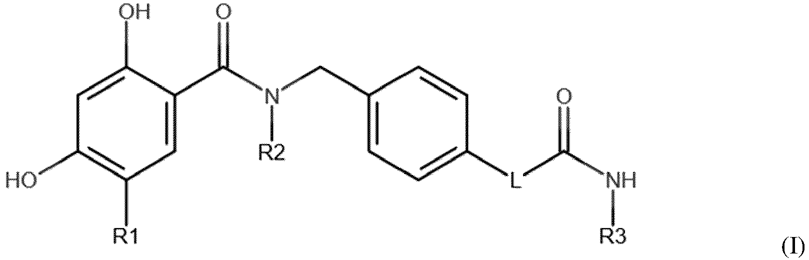 or pharmaceutically acceptable salts, solvates, hydrates, polymorphs, tautomers, stereoisomers, or isotopically enriched derivatives. The definitions and embodiments of the substitutions are as described herein.

The term "pharmaceutically acceptable salts" as used herein refers to compounds according to the disclosure used in the form of salts derived from inorganic or organic acids and bases. Included among acid salts, for example, are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectianate, persulfate, phenylproprionate, picrate, pivalate, propionate, succinate, tartrate, thiocyanate, tosylate and undecanoate. Salts derived from appropriate bases include alkali metal (e.g., sodium), alkaline earth metal (e.g., magnesium), ammonium and NW⁴⁺ (wherein W is C₁₋₄ alkyl).

As used herein, "prodrugs" are intended to include any covalently bonded carriers that release the active compound according to formula (I) through *in vivo* physiological action, such as hydrolysis, metabolism and the like, when such prodrug is administered to a subject. The suitability and techniques involved in making and using prodrugs are well known by a person of ordinary skill in the art. Prodrugs of the compounds of formula (I) (parent compounds) can be prepared by modifying functional groups present in the compounds in such a way that the modifications are cleaved, either in routine manipulation or *in vivo,* to the parent compounds. "Prodrugs" include the compounds of formula (I) wherein a hydroxy, amino, or sulfhydryl group is bonded to any group that, when the prodrugs are administered to a subject, cleaves to form a free hydroxyl, free amino, or free sulfhydryl group, respectively. Examples of prodrugs include, but are not limited to, derivatives and metabolites of the compounds of formula (I) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. In certain embodiments, prodrugs of the compounds of formula (I) with carboxyl functional groups are the lower alkyl (e.g., C₁-C₆) esters of the carboxylic acid. The carboxylate esters are conveniently formed by esterifying any of the carboxylic acid moieties present on the molecule.

The compounds of the disclosure can exist as solvates. As used herein and unless otherwise indicated, the term "solvate" refers to a compound of formula (I), or a pharmaceutically acceptable salt thereof, that further includes a stoichiometric or non-stoichiometric amount of a solvent bound by non-covalent intermolecular forces. If the solvent is water, the solvate may be conveniently referred to as a "hydrate," for example, a hemihydrate, a mono-hydrate, a sesqui-hydrate, a di-hydrate, a tri-hydrate, etc.

The term "tautomer" as used herein refers to compounds whose structures differ markedly in the arrangement of atoms, but which exist in easy and rapid equilibrium, and it is to be understood that compounds provided herein may be depicted as different tautomers, and when compounds have tautomeric forms, all tautomeric forms are intended to be within the scope of the invention, and the naming of the compounds does not exclude any tautomer. Exemplary tautomerizations include, but are not limited to, amide-to-imide; enamine-to-imine; enamine-to-(a different) enamine tautomerizations; and keto-to-enol.

The term "stereoisomers" refers to compounds that have identical chemical constitution,but differ with regard to the arrangement of the atoms or groups in space. Stereoisomers include diastereomers, enantiomers, conformers and the like.

The term "polymorph" refers to a crystalline form of a compound (or a salt, hydrate, or solvate thereof). All polymorphs have the same elemental composition. Different crystalline forms usually have different X-ray diffraction patterns, infrared spectra, melting points, density, hardness, crystal shape, optical and electrical properties, stability, and solubility. Recrystallization solvent, rate of crystallization, storage temperature, and other factors may cause one crystal form to dominate. Various polymorphs of a compound can be prepared by crystallization under different conditions.

As used herein, "isotopically enriched derivatives" refers to a compound containing at least one atom having an isotopic composition other than the natural isotopic composition of that atom. "Isotopic enrichment" can be expressed in terms of the percentage of incorporation of an amount of a specific isotope at a given atom in a molecule in the place of the atom's natural isotopic abundance.

The compound of the present disclosure may exist in one or more particular geometric, optical, enantiomeric, diasteriomeric, epimeric, atropic, stereoisomeric, tautomeric, conformational, or anomeric forms, including, but not limited to, *cis-* and *trans-*forms; E- and Z-forms; c-, t-, and r-forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof.

The compounds of the present invention can be prepared using methods known to those skilled in the art in view of this disclosure. For example, the preferred compounds of the invention can be prepared as shown in the following schemes:

### Synthesis Examples

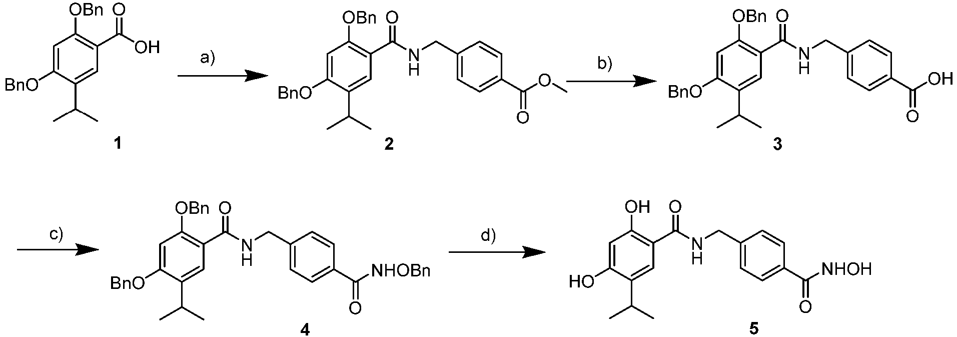

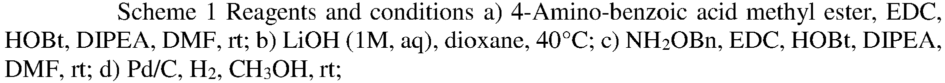

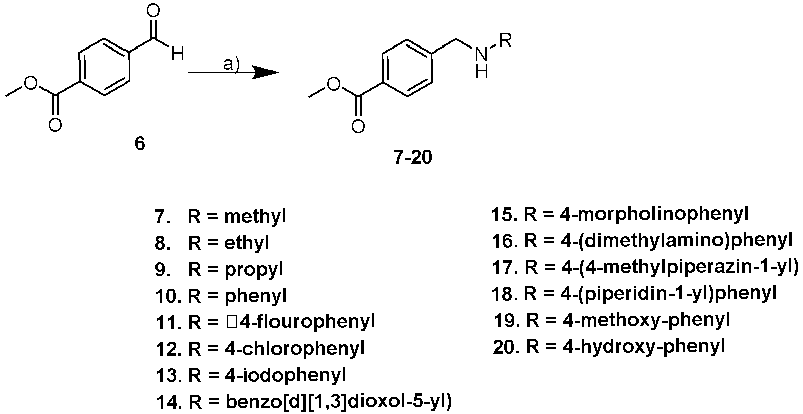

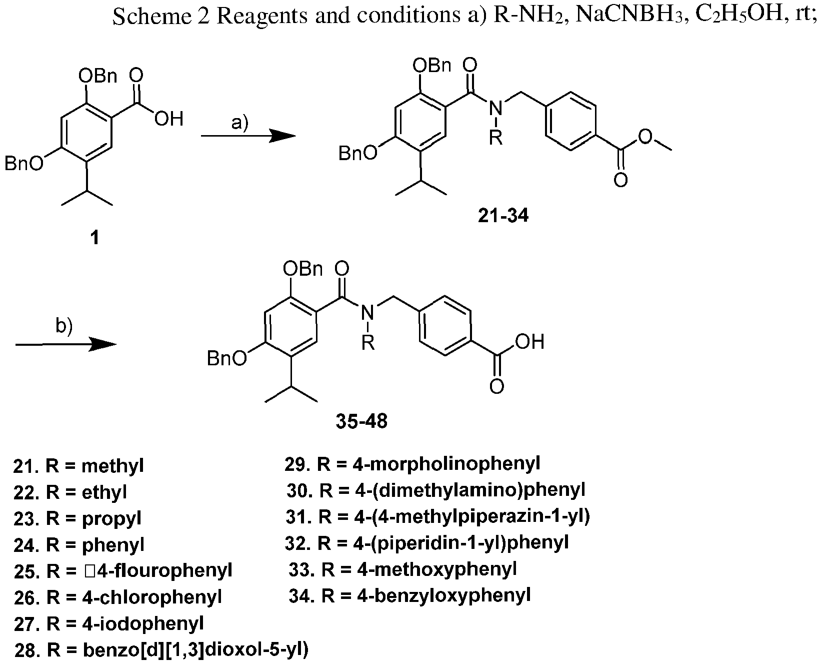

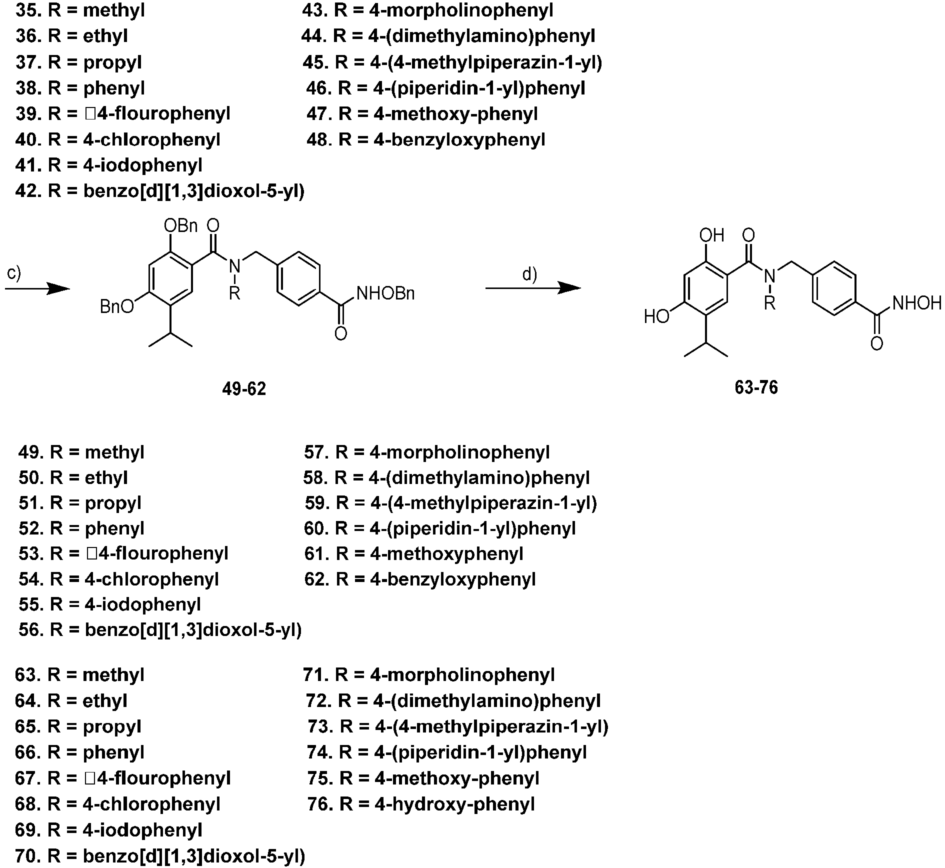

Scheme 2 Reagents and conditions a) i) Oxalyl chloride, DCM, rt; ii) 3,5-bis(benzyloxy)-2-isopropylbenzoic acid, TEA, DCM, 0°C, rt; b) LiOH (1M, aq), dioxane, 40°C; c) NH₂OBn, EDC, HOBt, DIPEA, DMF, rt; d) For **49-52** and **56-62,** Pd/C, H₂, CH₃OH, rt; for **53-55,** BCl₃ (1M in heptane), DCM.

Particular but non-limiting embodiments of the compounds of the present disclosure are listed below:
4-dihydroxy-N-(4-(hydroxycarbamoyl)benzyl)-5-isopropyl-N-methylbenzamide (compound 63), N-ethyl-2,4-dihydroxy-N-(4-(hydroxycarbamoyl)benzyl)-5-isopropylbenzamide (compound 64), 2,4-dihydroxy-N-(4-(hydroxycarbamoyl)benzyl)-5-isopropyl-N-propylbenzamide (compound 65), 2,4-dihydroxy-N-(4-(hydroxycarbamoyl)benzyl)-5-isopropyl-N-phenylbenzamide (compound 66), N-(4-fluorophenyl)-2,4-dihydroxy-N-(4-(hydroxycarbamoyl)benzyl)-5-isopropylbenzamide (compound 67), N-(4-chlorophenyl)-2,4-dihydroxy-N-(4-(hydroxycarbamoyl)benzyl)-5-isopropylbenzamide (compound 68), 2,4-dihydroxy-N-(4-(hydroxycarbamoyl)benzyl)-N-(4-iodophenyl)-5-isopropylbenzamide (compound 69), N-(benzo[d][1,3]dioxol-5-yl)-2,4-dihydroxy-N-(4-(hydroxycarbamoyl)benzyl)-5-isopropylbenzamide (compound 70), 2,4-dihydroxy-N-(4-(hydroxycarbamoyl)benzyl)-5-isopropyl-N-(4-morpholinophenyl)benzamide (compound 71), N-(4-(dimethylamino)phenyl)-2,4-dihydroxy-N-(4-(hydroxycarbamoyl)benzyl)-5-isopropylbenzamide (compound 72), 2,4-dihydroxy-N-(4-(hydroxycarbamoyl)benzyl)-5-isopropyl-N-(4-(4-methylpiperazin-1-yl)phenyl)benzamide (compound 73), 2,4-dihydroxy-N-(4-(hydroxycarbamoyl)benzyl)-5-isopropyl-N-(4-(piperidin- 1-yl)phenyl)benzamide (compound 74), 2,4-dihydroxy-N-(4-hydroxycarbamoyl-benzyl)-5-isopropyl-N-(4-methoxyphenyl)-benzamide (compound 75), and 2,4-dihydroxy-N-(4-hydroxycarbamoyl-benzyl)-N-(4-hydroxy-phenyl)-5-isopropyl-benzamide (compound 76).

The compound as described therein can be therapeutically administered as a neat chemical, but it may be useful to administer the compounds as part of a pharmaceutical composition or formulation. Thus, the present disclosure provides a pharmaceutical composition comprising a therapeutically effective amount of the bifunctional compound as described therein or pharmaceutically acceptable salts, tautomers, stereoisomers, solvates, hydrates, polymorphs, or isotopically enriched derivatives, and one or more pharmaceutically acceptable excipients.

The pharmaceutical compositions can be administered in a variety of dosage forms including, but not limited to, a solid dosage form or a liquid dosage form, an oral dosage form, a parenteral dosage form, an intranasal dosage form, a suppository, a lozenge, a troche, buccal, a controlled release dosage form, a pulsed release dosage form, an immediate release dosage form, an intravenous solution, a suspension or combinations thereof. The pharmaceutical compositions can be administered, for example, by oral or parenteral routes, including intravenous, intramuscular, intraperitoneal, subcutaneous, transdermal, airway (aerosol), rectal, vaginal and topical (including buccal and sublingual) administration.

An "excipient" generally refers to a substance, often an inert substance, added to a pharmacological composition or otherwise used as a vehicle to further facilitate administration of a compound. Examples of excipients include, but are not limited to, inert diluents, disintegrating agents, binding agents, lubricating agents, sweetening agents, flavoring agents, coloring agents, preservatives, effervescent mixtures, and adsorbents. Suitable inert diluents include, but are not limited to, sodium and calcium carbonate, sodium and calcium phosphate, lactose, and the like. Suitable disintegrating agents include, but are not limited to, starches, such as corn starch, cross-linked polyvinyl pyrrolidone, agar, alginic acid, or a salt thereof, such as sodium alginate, and the like. Binding agents may include, but are not limited to, magnesium aluminum silicate, starches such as corn, wheat or rice starch, gelatin, methylcellulose, sodium carboxymethylcellulose, polyvinylpyrrolidone, and the like. A lubricating agent, if present, will generally be magnesium stearate and calcium stearate, stearic acid, talc, or hydrogenated vegetable oils. If desired, the tablet may be coated with a material such as glyceryl monostearate or glyceryl distearate, to delay absorption in the gastrointestinal tract. The compositions can also be formulated as chewable tablets, for example, by using substances such as mannitol in the formulation.

The present disclosure also provides a method for amelioration and/or treatment of tumors in a subject in need of such amelioration and/or treatment, the method comprising administering the pharmaceutical composition as described herein to the subject.

The present disclosure also provides a method for removing immune suppression from tumor microenvironments or stimulating an immune system against tumors in a subject in need of such removal or stimulation, the method comprising administering the pharmaceutical composition as described herein to the subject.

It is believed, though not intended to be restricted by any theoretical limitations, that HDAC6 inhibitors are able to block the IFN γ-induced STAT1 pathway (Ginter et al., Cell Signal 24(7):1453-60, 2012*)* and further induce the infiltration of cytotoxic T cells in the TME *(*Falkenber et al., Nature Reviews Drug Discovery 13, 673-691, 2014*).* The present disclosure also provides a method for inhibiting HDAC6 in tumor microenvironments in a subject in need of such inhibition, the method comprising administering the pharmaceutical composition as described herein to the subject. Furthermore, in some embodiments of the disclosure, the method is for blocking signal transducer and activator of STAT1 pathway induced by IFN-γ. When tumor infiltrating lymphocytes (TILs) attack cancer cells, the cytohormone IFN-γ is produced, and tumor cells develop a self-protective mechanism by activating the STAT1 pathway in tumor cells to induce PD-L1 and IDO expression. IDO is an immunosuppressive protein that breaks down tryptophan into kynurenine, a cytoplasmic enzyme. However, tryptophan is an essential amino acid for T cells, wherein T cells are highly sensitive to tryptophan deficiency. Therefore, increased IDO activity and reduced tryptophan concentration are able to inhibit T cell proliferation and lead to T cell apoptosis. In some embodiments of the disclosure, the method is for lowering PD-L1 or IDO expression of the tumor. Furthermore, HDAC induces deacetylation of the lysine site of histone or other proteins, which affects transcription and translation functions and regulates gene expression. Cancer cells over-activate deacetylation, resulting in a decrease in the expression of tumor suppressor genes, which in turn promotes cancer cell growth. In some embodiments of the disclosure, the method is for inhibiting acetylation of α-tubulin and histone. Inhibition of HDAC increases the expression of the tumor suppressor gene, which affects the growth of cancer cells.

In some embodiments of the disclosure, the method further comprises administering a tumor specific inhibitor, for example, Rapamycin, Cabozantinib, and/or Erlotinib. For example, the inhibitor comprises a polypeptide, a small molecule inhibitor, RNA interference (RNAi), an antibody, or any fragment or combination thereof. In one aspect, the antibody or antibody fragment is partially humanized, fully humanized, or chimeric. Optionally, the antibody or antibody fragment comprises a nanobody, an Fab, an Fab', an (Fab')2, an Fv, a single-chain variable fragment (ScFv), a diabody, a triabody,a tetrabody, a Bis-scFv, a minibody, an Fab2, an Fab3 fragment, or any combination thereof. An example of such antibody is an anti-EGFR antibody.

In some embodiments of the disclosure, the method further comprises administering a chemotherapy drug. Chemotherapy drugs are divided into several groups based on their effect on cancer cells, the cellular activities or processes the drug interferes with, or the specific phases of the cell cycle the drug affects. Accordingly, chemotherapy drugs fall in one of the following categories: alkylating agents, antimetabolites, anthracyclines, topoisomerase I and II inhibitors, mitotic inhibitors, platinum based drugs, steroids and anti-angiogenic agents.

Examples of antimetabolites include purine antagonists, pyrimidine antagonists, and folate antagonists. Specific examples of antimetabolites include 5-fluorouracil (also known as 5FU), capecitabine, 6-mercaptopurine, methotrexate, gemcitabine, cytarabine, fludarabine and pemetrexed. Platinum-based chemotherapeutic drugs include cisplatin (also known as cisplatinum or cis-diamminedichloridoplatinum II (CDDP), carboplatin and oxaliplatin. Examples of mitotic inhibitors include paclitaxel, docetaxel, etoposide, vinblastine and vincristine. Anthracycline antibiotics, for example, include, daunorubicin, doxorubicin (also known as Adriamycin^{®} and doxorubicin hydrochloride), respinomycin D and idarubicin. Alkylating antineoplastic agents act nonspecifically. Cyclophosphamide is an alkylating agent; however, it is a highly potent immunosuppressive substance. Examples of topoisomerase I inhibitors include topotecan and irinotecan. Examples of topoisomerase II inhibitors include etoposide and teniposide. Non-limiting examples of anti-angiogenic agents include the monoclonal antibody bevacizumab, dopamine and tetrathiomolybdate.

In some embodiments of the disclosure, the method further comprises administering an immunotherapy drug. In some embodiments of the disclosure, the method further comprises administering an immune checkpoint inhibitor, wherein "checkpoint inhibitor" refers to any agent blockading immune system inhibitory checkpoints. Examples of the checkpoint inhibitor include, but are not limited to, an inhibitor of CTLA-4, PD-1, PD-L1, PD-L2, LAG-3, BTLA, B7H3, B7H4, TIM3, KIR, or A2aR. In certain aspects, the at least one immune checkpoint inhibitor is a human PD-1 axis-binding antagonist. In some aspects, the PD-1 axis-binding antagonist is selected from the group consisting of a PD-1 binding antagonist, a PD-L1-binding antagonist and a PDL2-binding antagonist. In certain aspects, the PD-1 axis-binding antagonist is a PD-1-binding antagonist. In some aspects, the PD-1-binding antagonist inhibits the binding of PD-1 to PD-L1 and/or PDL2. In particular aspects, the PD-1-binding antagonist is a monoclonal antibody or antigen binding fragment thereof. In specific aspects, the PD-1-binding antagonist is nivolumab, pembrolizumab (e.g., KEYTRUDA^{®}), pidillizumab, AMP-514, REGN2810, CT-011, BMS 936559, MPDL3280A or AMP-224. In some aspects, the at least one immune checkpoint inhibitor is an anti-CTLA-4 antibody. In particular aspects, the anti-CTLA-4 antibody is tremelimumab, or ipilimumab (e.g., YERVOY^{®}). In certain aspects, the at least one immune checkpoint inhibitor is an anti-killer-cell immunoglobulin-like receptor (KIR) antibody. In some aspects, the anti-MR antibody is lirilumab.

In some embodiments of the disclosure, the compound as disclosed herein and the immune checkpoint inhibitor, tumor-targeting inhibitor, or chemotherapy drug are each formulated as single medicaments for simultaneous, separate or sequential administration. In some embodiments of the disclosure, the compound as disclosed herein and the immune checkpoint inhibitor, tumor-targeting inhibitor, or chemotherapy drug are co-administered simultaneously, separately or sequentially or co-administered in combination as a coformulation. As used herein, the term "combination," "therapeutic combination" or "pharmaceutical combination," as used herein, defines either a fixed combination in one dosage unit form or a kit of parts for the combined administration where Compound A and Compound B may be administered independently at the same time or separately within time intervals. As used herein, the term "co-administration" or "combined administration" is defined to encompass the administration of the selected therapeutic agents to a single patient, and is intended to include treatment regimens in which the agents are not necessarily administered by the same route of administration or at the same time.In some embodiments of the disclosure, therapeutic combination has synergistic properties greater than the properties of each of the compounds as disclosed herein and the immune checkpoint inhibitor, tumor-targeting inhibitor, or chemotherapy drug.

The present disclosure also provides a method for inducing infiltration of cytotoxic T cells in tumor microenvironments in a subject in need of such induction, the method comprising administering the pharmaceutical composition as described herein to the subject. In some embodiments of the disclosure, the method is for inducing granzyme B expression.

It is believed, though not intended to be restricted by any theoretical limitations, that HSP90 plays an important role in regulating the correct conformation and stability of proteins, including stabilizing many proteins required for tumor growth, enhancing tumor survival and resistance to the immune system, and promoting cancer cell growth (Trepel et al., Nature Reviews Cancer 10, 537- 549, 2010)*.* The present disclosure provides a method for inhibiting HSP90 in tumor microenvironments in a subject in need of such inhibition, the method comprising administering the pharmaceutical composition as described herein to the subject. In some embodiments of the disclosure, the method is for destabilizing proteins for tumor growth. In some embodiments of the disclosure, the method is for increasing HSP70 expression. Furthermore, in some embodiments of the disclosure, the method is for reducing expression of Src, AKT, Rb or FAK.

The present disclosure provides a method for inhibiting tumor growth and/or inhibiting tumor recurrence in a subject in need of such inhibition, the method comprising administering the pharmaceutical composition as described herein to the subject. In some embodiments of the disclosure, the tumor is a solid tumor. Examples of the tumor include, but are not limited to, colorectal cancer, pancreatic carcinoma, small cell lung cancer, non-small cell lung cancer, renal cell carcinoma, breast cancer, head and neck cancer, prostate cancer, malignant gliomas, osteosarcoma, gastric cancer, malignant mesothelioma, multiple myeloma, ovarian cancer, synovial sarcoma, thyroid cancer, or melanoma.

The present disclosure also provides a method for reducing the level of regulatory T cells (also called Tregs or Treg cells) in a subject in need of such reduction, the method comprising administering the pharmaceutical composition as described herein to the subject. The regulatory T cells are immunosuppressive and assist in tumor immune escape.

The present disclosure also provides a method for increasing central memory T cell level in a subject in need of such increase, the method comprising administering the pharmaceutical composition as described herein to the subject. The central memory T cells inhibit the recurrence of cancer.

It is known that the clinical efficacy of monotherapy with anti-PD1 antibodies is limited, with only 20-25% of patients treated effectively. Due to the complexity of the tumor microenvironment, the interaction between the cancer and the immune system has many factors, such as the inability of cytotoxic T cells to infiltrate the tumor area and immunosuppressive proteins, resulting in limited therapeutic efficacy. Therefore, a strategy of drug administration alone may yield better clinical outcomes than a combination of multiple drugs.

In order for the invention described herein to be more fully understood, the following examples are set forth. The examples described in this application are offered to illustrate the compounds, pharmaceutical compositions, and methods provided herein and are not to be construed in any way as limiting their scope.

### EXAMPLES

### Materials and Methods

### Chemistry

The chemical properties of the HSP90/HDAC6 dual inhibitors as described in Synthesis Examples are evaluated. Bruker DRX-300 spectrometer operating at 300 MHz was used to obtain the nuclear magnetic resonance spectra. 1H NMR spectra were recorded at 300 MHz and the 13C NMR spectra were recorded at 75 MHz. JEOL (JMS-700) electrospray ionization (ESI) mass spec-trometer was used to obtain high resolution mass spectra (HRMS). Shimadzu LC-2030C LT was used to determine the purity of the final compounds. Silica gel (Merck Kieselgel 60, No. 9385) was used for the column chromatography. The characterization data of the compounds has been placed in the supporting information.

### Biology

### Cell culture

The effects on cells of the HSP90/HDAC6 dual inhibitors as described in Synthesis Examples (such as Compounds 63, 64, and 66 to 76) were evaluated. HCT116 cells were cultured in McCoy's 5a medium supplemented with 10% FBS. LS174T cells were cultured in MEM medium with 10% FBS. CT26 cells were cultured in DMEM medium with 10% CCS. All culture medium contained 1% penicillin-streptomycin, and all cells lines were cultured in a cell incubator with 5% CO₂ at 37 °C.

### HDAC enzymes inhibition assays

The inhibitory effect of the HSP90/HDAC6 dual inhibitors as described in Synthesis Examples (such as Compounds 63, 64, and 66 to 76) on HDAC enzymes was evaluated using enzyme inhibition assays performed by Reaction Biology Corporation, Malvern, PA. The substrate for HADC 1, 3, and 6 was a fluorogenic peptide derived from the p53 residues 379-382 (RHKK(Ac)). All compounds were dissolved in DMSO and tested in at least 10-dose IC₅₀ mode with 3-fold serial dilution starting at 10 µM. The IC₅₀ values were the result of a single experiment. Trichostatin A (TSA) was used as the reference.

### HSP90 inhibition assays

The inhibitory effect of the HSP90/HDAC6 dual inhibitors as described in Synthesis Examples (such as Compounds 63, 64, and 66 to 76) on HSP90 was evaluated using HSP90 inhibition assays performed by Reaction Biology Corporation, Malvern, PA. The assay is based on the competition of fluorescently labeled geldanamycin (FITC-GM) for binding to HSP90. FITC-GM binds to the ATP-binding pocket of HSP90; thus, ATP competitive inhibitors are identified through this experimental assay. The assay procedure involved the addition of compounds (dissolved in DMSO) to the HSP solution by using acoustic technology followed by incubation for 30 min. FITC-GM was then added, and the solution was incubated for 3 h. Finally, fluorescence polarization was measured and mP was calculated.

### MTT assay

The effects on cell viability of the HSP90/HDAC6 dual inhibitors as described in Synthesis Examples (such as Compounds 63, 64, and 66 to 76) were evaluated. Cells were seeded in 96-well plates (5000 cells/well). The next day, the serially diluted drugs were added into the wells and incubated at 37 °C for 48 h. Cell viability was measured by the MTT assay and is expressed as percentages of surviving drug-treated cells relative to untreated control cells. The IC₅₀ was calculated by non-linear regression analysis.

### Immunoblotting analysis

The effects of the HSP90/HDAC6 dual inhibitors as described in Synthesis Examples (such as Compounds 63, 64, and 66 to 76) on several factors were evaluated. Cells were lysed in RIPA buffer (Thermo Fisher Scientific, Wal-tham, MA, USA) and protease inhibitor (Roche, Basel, CH). Sample were further separated in 8% or 15% SDS-PAGE and transferred to nitrocellulose membrane and blocked with 5% skim milk (w/v in PBS). Nitrocellulose membranes were then incubated with primary antibodies against the following antigens: HSP90, Src, AKT, Rb, phosphorylated Rb (p-Rb), FAK, HSP70, α-tubulin acetylation, acetyl histone H3, phosphorylated STAT1 (p-STAT1), STAT1 (Cell signaling, Danvers, MA, USA), α-tubulin, Histone H3 (Genetex, Irvine, USA), IDO (Santa Cruz Biotechnology Dallas, TX, USA) and PD-L1 (Novus, Littleton, CO, USA) at 4°C for overnight. The target proteins were visualized by using HRP-conjugated secondary antibodies (Jackson ImmunoResearch Laboratories, West Grove, PA, USA) and enhanced chemiluminescence kit (Thermo Fisher Scientific, Waltham, MA, U.S.A.).

### Analysis of PD-L1 expression on cell surface by flow cytometry

The effects of the HSP90/HDAC6 dual inhibitors as described in Synthesis Examples (such as Compounds 63, 64, and 66 to 76) on PD-L1 expression were evaluated. Cells were seeded in 6-well plates (3.2 x 10⁵ cells/well) and treated on the next day with IFN-γ and compounds or DMSO (vehicle) at indicated concentrations for 48 h. The treated cells were suspended (3x10⁵ cells/tube) and stained with PD-L1 antibody (Invitrogen, Waltham, USA) and FITC-conjugated secondary antibodies on ice for 1 h. The fluorescence signal of the cells was analyzed by FASCalibur flow cytometer and CellQuest software (BD Biosciences, San Jose, CA, USA).

### Syngeneic tumor studies

The effects of the HSP90/HDAC6 dual inhibitors as described in Synthesis Examples (such as Compounds 63, 64, and 66 to 76) on syngeneic tumor treatment were evaluated. Female BALB/c mice were subcutaneously implanted with mouse colorectal cancer CT26 cells (2 x 10⁵ cells/mouse) in the left unilateral back. After tumor size reached 50 mm³, mice were i.v. injected with compound 17, STA9090 (Ganetespib), or anti-PD-1 antibody (clone RMP1-14) (4-8 mice per group). The progression of tumor sizes and body weights was measured twice per week. Tumor volume formula: V (mm³) = (length x width x height)/2. Mice were sacrificed on day 25, and tumors and blood were collected for the analysis of immune cell populations.

### Immunohistochemistry (IHC) staining

The effects of the HSP90/HDAC6 dual inhibitors as described in Synthesis Examples (such as Compounds 63, 64, and 66 to 76) on several factors were evaluated. IHC-Paraffin: Tumor tissues were excised from the mice, fixed in 4% formaldehyde solution in PBS, and embedded in paraffin. Sections of 4-5 µm were cut and placed in an oven at 60°C for overnight. After deparaffinizing and rehydrating the section, the antigen retrieval was performed with citrate buffer (10 mM citrate acid, pH 6) or Tris buffer (10 mM Tris, 1 mM EDTA, pH9) by autoclave for 30 min at 121C. The sections were blocked with 10% goat serum and then incubated with primary antibodies against the following antigens: IDO (Santa Cruz Biotechnology Dallas, TX, USA), CD8 (Novus, Littleton, CO, USA) and granzyme B (Abcam, Cambridge, UK) at 4 C for overnight. IHC- Frozen for PD-L1: Tumor tissues were collected from the mice, embedded in OCT compound (Sakura Finetek, Torrance, CA, U.S.A.). Sections of 10 µm were cut, fixed with 100% acetone for 10 min, and blocked with 10% goat serum. The slides were incubated with PD-L1 primary antibodies at 4 C for overnight. Then, the slides were added in 0.3 % hydrogen peroxide (H₂O₂) for 10 min to quench endogenous peroxidase activity. The slides were further incubated with HRP polymer - conjugated secondary antibodies (R&D system, Minneapolis, MN, USA) at room temperature for 1 h. Color development was per-formed according to the DAB substrate kit (Dako, Glostrup, Denmark) and nuclei were counterstained with hematoxylin. Images of the tumor tissues sections were captured by Mirax Scan (CARL ZEISS, Germany) or TissueFAXS (Vienna, Austria) and analyzed by HistoQuest software (Vienna, Austria).

### Analysis of the immune cell infiltration of tumor cells by flow cytometry

The effects of the HSP90/HDAC6 dual inhibitors as described in Synthesis Examples (such as Compounds 63, 64, and 66 to 76) on immune cell filtration of tumor cells were evaluated. Tumor tissues were excised from the mice, and cut into small pieces with a scalpel. Cells in tumor stroma were isolated according to the MACS tumor dissociation kit (Miltenyi Biotec, Bergisch Gladbach, Germany) and filtered through a 70 µm cell strainer. Red blood cells were removed by using ACK lysing buffer (Gibco, Life Technologies, Eugene, OR, U.S.A.). Then, cells were stained with the indicated fluorescent labeled antibodies for 1 h. For staining intracellular molecules, cells were fixed and permeabilized according to the FOXP3/Transcrip- tion factor staining buffer kit (Tonbo Biosciences, San Diego, CA, U.S.A.) and were stained with fluorescently labeled antibodies for 1 h. The fluorescence signal of the cells was detected by flow cytometer (Sony SA3800 San Jose, CA, U.S.A.).

### Statistical analysis

Data was analyzed by using GraphPad Prism software (San Diego, CA, USA). The t-test (two-tailed) was used to determine if there is a statistical difference between the mean values. A P value<0.05 is considered significant.

### Synthesis Examples

### Example 1 Synthesis of 4-[(2,4-Bis-benzyloxy-5-isopropyl-benzoylamino)-methyl]-benzoic acid methyl ester (2)

A mixture of compound 1 (500 mg, 1.32 mmol), EDC.HCl (509 mg, 2.65 mmol), HOBt (267 mg, 1.98 mmol) and DIPEA (0.574 mL, 3.30 mmol) in DMF (5 mL) was stirred at room temperature for 30 min before adding 4-Amino-benzoic acid methyl ester (199 mg, 1.32 mmol). After being stirred for a further 5 h, the reaction mixture was quenched with water and extracted with EtOAc (50 mL x 3). The combined organic layer was dried over anhydrous MgSO₄ and concentrated under reduced pressure. The residue was purified by silica gel chromatography (hexane:EtOAc: 3 : 2) to give a semisolid product. The resulting product was dissolved in CH₃OH (10 mL). A catalytic amount of 10% palladium on carbon was added and the reaction mixture was stirred for 24 hours under hydrogen. The reaction mixture was filtered over celite and the filtrate was dried in vacuum to afford 2 in 82% yield. ¹H NMR (300 MHz, CDCl₃): 1.15 (d, J = 6.9 Hz, 6H), 3.25 (m, 1H), 3.89 (s, 3H) 4.72 (s, 2H), 5.01 (s, 2H), 5.11 (s, 2H), 6.34 (s, 1H), 7.07 (s, 1H), 7.31 - 7.44 (m, 12H), 7.71 (d, J = 8.7 Hz, 2H).

### Example 2 Synthesis of 4-[(2,4-Bis-benzyloxy-5-isopropyl-benzoylamino)-methyl]-benzoic acid (3)

A mixture of compound **2** (600 mg, 1.14 mmol), 1 M LiOH(aq) (7 ml) and dioxane (15 mL) was stirred at 40 °C for 2 h. The reaction was concentrated under reduced pressure and water was added. The mixture was acidified with 3 N HCl and extracted with ethyl acetate (50 mL x 3). The combined organic layer was dried over anhydrous MgSO₄ and concentrated under reduced pressure to yield the acid **3** in 96% yield; ¹H NMR (300 MHz, CD₃OD): 1.11 (d, J = 6.9 Hz, 6H), 3.21 (m, 1H), 4.79 (s, 2H), 5.08 (s, 2H), 5.13 (s, 2H), 6.38 (s, 1H), 7.17 (s, 1H), 7.35 - 7.42 (m, 12H), 7.75 (d, J = 8.7 Hz, 2H).

### Example 3 Synthesis of 2,4-Bis-benzyloxy-N-(4-benzyloxycarbamoyl-benzyl)-5-isopropyl-benzamide (4)

A mixture of compound **3** (500 mg, 0.98 mmol), EDC.HCl (374 mg, 1.96 mmol), HOBt (198 mg, 1.47 mmol) and DIPEA (0.42 mL, 2.44 mmol) in DMF (5 mL) was stirred at room temperature for 30 min before adding NH₂OBn.HCl (156 mg, 0.98 mmol). After being stirred for a further 5 h, the reaction mixture was quenched with water and extracted with EtOAc (50 mL x 3). The combined organic layer was dried over anhydrous MgSO₄ and concentrated under reduced pressure. The residue was purified by silica gel chromatography (EtOAc) to give compound **4** in 75 % yield. ¹H NMR (300 MHz, CD₃OD): 1.12 (d, J = 6.9 Hz, 6H), 3.11 (m, 1H), 4.79 (s, 2H), 5.08 (s, 2H), 5.13 (s, 4H), 6.38 (s, 1H), 7.14 (s, 1H), 7.31 - 7.41 (m, 17H), 7.71 (d, J = 8.7 Hz, 2H).

### Example 4 Synthesis of 2,4-dihydroxy-N-(4-hydroxycarbamoyl-benzyl)-5-isopropyl-benzamide (5)

To the solution of compound 4 (200 mg, 0.32 mmol) in CH₃OH (10 mL), a catalytic amount of 10% palladium on carbon was added and the reaction mixture was stirred for 24 hours under hydrogen. The reaction mixture was filtered over celite and the filtrate was dried in vacuum to obtain a residue that was purified by silica gel chromatography (EtOAc:CH3OH: 9.5:0.5) to give compound **5** in 62 % yield **5;** ¹H NMR (300 MHz, CD₃OD): 1.24 (d, J = 6.9 Hz, 6H), 3.18 (m, 1H), 4.64 (s, 2H), 6.32 (s, 1H), 7.46 (d, J = 8.1 Hz, 2H), 7.61 (s, 1H), 7.87 (d, J = 8.4 Hz, 2H). HRMS (ESI) for C₂₆H₂₁ClN₈O₃ (M+H⁺): calcd, 345.1450; found, 345.1450.

### Example 5 Synthesis of methyl 4-((methylamino)methyl)benzoate (7)

To the solution of compound **6** (500 mg, 3.04 mmol) in methanol (10 ml), methyl amine (40 wt. % in H₂O, 1 mL) was added. The reaction mixture was stirred at room temperature for 1 h and then sodium borohydride (172 mg, 4.56 mmol) was added at 0 °C. The reaction mixture was quenched with water and extracted with EtOAc (20 mL x 3). The combined organic layer was dried over anhydrous MgSO₄ and concentrated under reduced pressure to give 7 in 92 % yield. ¹H NMR (300 MHz, CDCl₃): 2.48 (s, 3H), 3.83 (s, 2H), 3.94 (s, 3H), 7.42 (d, J = 8.7 Hz, 2H), 8.02 (d, J = 8.4 Hz, 2H).

### Example 6 Synthesis of methyl 4-((ethylamino)methyl)benzoate (8)

The title compound **8** was obtained in 94% yield from compound **6** using ethylamine in a manner similar to that described for the synthesis of compound **7.** ¹H NMR (300 MHz, CDCl₃) :1.16 (t, J = 7.2 Hz, 3H), 2.70 (q, J = 7.2 Hz, 2H), 3.87 (s, 2H), 3.93 (s, 3H), 7.41 (d, J = 8.7 Hz, 2H), 8.01 (d, J = 8.4 Hz, 2H).

### Example 7 Synthesis of methyl 4-((propylamino)methyl)benzoate (9)

The title compound **9** was obtained in 91% yield from compound **6** using propylamine in a manner similar to that described for the synthesis of compound **7.** ¹H NMR (300 MHz, CDCl₃) :0.94 (t, J = 7.5 Hz, 3H), 1.56 (m, 2H), 2.63 (t, J = 7.5 Hz, 2H), 3.85 (s, 2H), 3.92 (s, 3H), 7.40 (d, J = 8.7 Hz, 2H), 8.01 (d, J = 8.1 Hz, 2H).

### Example 8 Synthesis of methyl 4-((phenylamino)methyl)benzoate (10)

To the solution of compound **6** (500 mg, 3.04 mmol) in ethanol (20 mL), aniline (283 mg, 3.04 mmol) and a few drops of glacial acetic acid were added. The reaction mixture was stirred at room temperature for 1 h before adding sodium cyanoborohydride (286 mg, 4.56 mmol). The reaction mixture was refluxed for 2h and then quenched with water. Ethyl acetate (20 mL x 3) was used for extraction. The combined organic layer was dried over anhydrous MgSO₄ and concentrated under reduced pressure. The residue was purified by silica gel chromatography (hexane:EtOAc::3: 2) to give a **10** in 78 % yield. ¹H NMR (300 MHz, CDCl₃) 3.81 (s, 3H), 4.79 (s, 2H), 6.79 - 6.83 (m, 3H), 7.13 (d, J = 7.2 Hz, 2H), 7.25 (d, J = 8.4 Hz, 2H), 7.64 (d, J = 8.1 Hz, 2H).

### Example 9 Synthesis of methyl 4-(((4-fluorophenyl)amino)methyl)benzoate (11)

The title compound **11** was obtained in 64% yield from compound **6** using 4-fluoroaniline in a manner similar to that described for the synthesis of compound **10.** ¹H NMR (300 MHz, CD₃OD): 3.98 (s, 3H), 4.44 (s, 2H), 6.49 (d, J = 8.1 Hz, 2H), 7.32 (d, J = 8.7 Hz, 2H), 7.40 (d, J = 8.7 Hz, 2H), 8.00 (d, J = 8.4 Hz, 2H).

### Example 10 Synthesis of methyl 4-(((4-chlorophenyl)amino)methyl)benzoate (12)

The title compound **12** was obtained in 68% yield from compound **6** using 4-chloroaniline in a manner similar to that described for the synthesis of compound **10.** ¹H NMR (300 MHz, CD₃OD): 3.92 (s, 3H), 4.54 (s, 2H), 6.48 (d, J = 8.7 Hz, 2H), 7.41 (d, J = 8.7 Hz, 2H), 7.43 (d, J = 8.7 Hz, 2H), 8.03 (d, J = 8.4 Hz, 2H).

### Example 11 Synthesis of methyl 4-(((4-iodophenyl)amino)methyl)benzoate (13)

The title compound **13** was obtained in 71% yield from compound **6** using 4-iodoaniline in a manner similar to that described for the synthesis of compound **10.** ¹H NMR (300 MHz, CD₃OD): 3.94 (s, 3H), 4.41 (s, 2H), 6.42 (d, J = 8.7 Hz, 2H), 7.43 (d, J = 8.7 Hz, 4H), 8.05 (d, J = 8.4 Hz, 2H).

### Example 12 Synthesis of methyl 4-((benzo[d][1,3]dioxol-5-ylamino)methyl)benzoate (14)

The title compound **14** was obtained in 79% yield from compound **6** using benzo[d][1,3]dioxol-5-amine in a manner similar to that described for the synthesis of compound **10.** ¹H NMR (300 MHz, CDCl₃): 3.84 (s, 3H), 4.97 (s, 2H), 5.99 (s, 2H), 6.52 (dd, J = 2.1 and 8.1 Hz, 1H), 6.69 (d, J = 2.1 Hz, 1H), 6.78 (d, J = 8.4 Hz, 1H), 7.39 (d, J = 8.1 Hz 2H), 7.65 (d, J = 8.1 Hz, 2H).

### Example 13 Synthesis of methyl 4-(((4-morpholinophenyl)amino)methyl)benzoate (15)

The title compound **15** was obtained in 77% yield from compound **6** using 4-morpholinoaniline in a manner similar to that described for the synthesis of compound **10.** ¹H NMR (300 MHz, CDCl₃) : 3.15 (t, J = 4.8 Hz, 4H), 3.78 (t, J = 4.5 Hz, 4H), 3.93 (s, 3H), 4.98 (s, 2H), 6.86 (d, J = 9.0 Hz, 2H), 6.93 (d, J = 9.0 Hz, 2H), 7.34 (d, J = 8.1 Hz, 2H), 7.75 (d, J = 8.1 Hz, 2H).

### Example 14 Synthesis of methyl 4-(((4-(dimethylamino)phenyl)amino)methyl)benzoate (16)

The title compound **16** was obtained in 72% yield from compound **6** using N1,N1-dimethylbenzene-1,4-diamine in a manner similar to that described for the synthesis of compound **10.** ¹H NMR (300 MHz, CDCl₃): 2.91 (s, 3H), 2.98 (s, 3H), 3.86 (s, 3H), 4.98 (s, 2H), 6.68 (d, J = 7.2 Hz, 2H), 6.87 (d, J = 6.9 Hz, 2H), 7.45 (d, J = 7.2 Hz, 2H), 7.62 (d, J = 8.4 Hz, 2H).

### Example 15 Synthesis of methyl 4-(((4-(4-methylpiperazin-1-yl)phenyl)amino)methyl)benzoate (17)

The title compound **17** was obtained in 74% yield from compound **6** using 4-(4-methylpiperazin-1-yl)aniline in a manner similar to that described for the synthesis of compound **10.** ¹H NMR (300 MHz, CD₃OD): 2.65 (s, 3H), 2.97 (bs, 4H), 3.12 (bs, 4H), 3.90 (s, 3H), 4.39 (s, 2H), 6.61 (d, J = 9.0 Hz, 2H), 6.87 (d, J = 9.0 Hz, 2H), 7.49 (d, J = 7.8 Hz, 2H), 7.97 (d, J = 8.1 Hz, 2H).

### Example 16 Synthesis of methyl 4-(((4-(piperidin-1-yl)phenyl)amino)methyl)benzoate (18)

The title compound **18** was obtained in 74% yield from compound **6** using 4-(piperidin-1-yl)aniline in a manner similar to that described for the synthesis of compound **10.** ¹H NMR (300 MHz, CD₃OD): 1.59 - 1.72 (m, 6H), 3.09 (bs, 4H), 3.91 (s, 3H), 4.37 (s, 2H), 6.68 (d, J = 9.0 Hz, 2H), 6.89 (d, J = 9.0 Hz, 2H), 7.56 (d, J = 7.8 Hz, 2H), 7.91 (d, J = 8.1 Hz, 2H).

### Example 17 Synthesis of methyl 4-(((4-methoxyphenyl)amino)methyl)benzoate (19)

The title compound **19** was obtained in 85% yield from compound **6** using 4-methoxyaniline in a manner similar to that described for the synthesis of compound **10.** ¹H NMR (300 MHz, CD₃OD): 3.79 (s, 3H), 3.85 (s, 3H), 4.98 (s, 2H), 6.85 (d, J = 9.0 Hz, 2H), 6.91 (d, J = 9.0 Hz, 2H), 7.38 (d, J = 8.4 Hz, 2H), 7.73 (d, J = 8.4 Hz, 2H).

### Example 17 Synthesis of methyl 4-(((4-(benzyloxy)phenyl)amino)methyl)benzoate (20)

The title compound **20** was obtained in 81% yield from compound **6** using 4-(benzyloxy)aniline in a manner similar to that described for the synthesis of compound **10.** ¹H NMR (300 MHz, CD₃OD): 3.83 (s, 3H), 4.89 (s, 2H), 5.05 (s, 2H), 6.61 (d, J = 9.0 Hz, 2H), 6.82 (d, J = 8.7 Hz, 2H), 7.25 - 7.431 (m, 7H), 7.85 (d, J = 8.4 Hz, 2H).

### Example 18 Synthesis of methyl 4-((2,4-bis(benzyloxy)-5-isopropyl-N-methylbenzamido)methyl)benzoate (21)

2,4-bis(benzyloxy)-5-isopropylbenzoic acid (**1**) (500 mg, 1.32 mmol) was dissolved in dry DCM (10 mL) and oxalyl chloride (1 mL) was added to the solution. The reaction mixture was stirred for 2h at room temperature. Solvent was evaporated under reduced pressure and the residue was again dissolved in dry DCM (10 mL). Compound **7** (236 mg, 1.32 mmol) was then added to the solution followed by addition of trimethylamine (0.46 ml, 3.32 mmol) at 0 °C. After being stirred for a further 5h at room temperature, the reaction mixture was quenched with water and extracted with EtOAc (50 mL x 3). The combined organic layer was dried over anhydrous MgSO₄ and concentrated under reduced pressure. The residue was purified by silica gel chromatography (hexane:EtOAc::1:1) to give compound **21** in 78 % yield. ¹H NMR (300 MHz, CDCl₃): 1.13 (d, J = 6.9 Hz, 6H), 2.89 (s, 3H), 3.23 (m, 1H), 3.89 (s, 3H) 4.79 (s, 2H), 4.99 (s, 2H), 5.12 (s, 2H), 6.34 (s, 1H), 7.07 (s, 1H), 7.35 - 7.41 (m, 12H), 7.72 (d, J = 8.4 Hz, 2H).

### Example 19 Synthesis of methyl 4-((2,4-bis(benzyloxy)-N-ethyl-5-isopropylbenzamido)methyl)benzoate (22)

The title compound **22** was obtained in 84% yield from compound **8** using compound 1 in a manner similar to that described for the synthesis of compound **21.** ¹H NMR (300 MHz, CDCl₃): 1.16 (d, J = 6.3 Hz, 6H), 1.25 (t, J = 5.4 Hz, 3H), 3.19 (m, 1H), 3.39 (q, J = 5.4 Hz, 2H), 3.91 (s, 3H), 4.79 (s, 2H), 5.01 (s, 4H), 6.49 (s, 1H), 6.99 (s, 1H), 7.32 - 7.49 (m, 12H), 7.79 (d, J = 8.1 Hz, 2H).

### Example 20 Synthesis of methyl 4-((2,4-bis(benzyloxy)-5-isopropyl-N-propylbenzamido)methyl)benzoate (23)

The title compound **23** was obtained in 78% yield from compound **9** using compound **1** in a manner similar to that described for the synthesis of compound **21.** ¹H NMR (300 MHz, CDCl₃): 0.92 (t, J = 7.5 Hz, 3H), 1.13 (d, J = 6.6 Hz, 6H), 1.76 (m, 2H), 3.21 (m, 1H), 3.32 (t, J = 7.5 Hz, 2H), 3.83 (s, 3H), 4.71 (s, 2H), 5.12 (s, 4H), 6.32 (s, 1H), 6.91 (s, 1H), 7.35 - 7.48 (m, 12H), 7.71 (d, J = 8.1 Hz, 2H).

### Example 21 Synthesis of methyl 4-((2,4-bis(benzyloxy)-5-isopropyl-N-phenylbenzamido)methyl)benzoate (24)

The title compound **24** was obtained in 75 % yield from compound **10** using compound **1** in a manner similar to that described for the synthesis of compound **21.** ¹H NMR (300 MHz, CDCl₃): 0.85 (d, J = 6.6 Hz, 6H), 2.95 (m, 1H), 3.81 (s, 3H), 4.79 (s, 2H), 5.21 (s, 4H), 6.34 (s, 1H), 6.72 (s, 1H), 7.13 (d, J = 7.2 Hz, 2H), 7.19 (t, J = 6.6 Hz, 1H), 7.21 - 7.42 (m, 14H), 7.64 (d, J = 8.1 Hz, 2H).

### Example 22 Synthesis of methyl 4-((2,4-bis(benzyloxy)-N-(4-fluorophenyl)-5-isopropylbenzamido)methyl)benzoate (25)

The title compound **25** was obtained in 67 % yield from compound **11** using compound **1** in a manner similar to that described for the synthesis of compound **21.** ¹H NMR (300 MHz, CDCl₃): 1.13 (d, J = 6.9 Hz, 6H), 3.24 (m, 1H), 3.94 (s, 3H), 4.98 (s, 2H), 5.12 (s, 4H) 6.31 (s, 1H), 6.72-6.75 (m, 4H), 7.06 (s, 1H), 7.34 - 7.44 (m, 12H), 7.85 (d, J = 8.1 Hz, 2H).

### Example 23 Synthesis of methyl 4-((2,4-bis(benzyloxy)-N-(4-chlorophenyl)-5-isopropylbenzamido)methyl)benzoate (26)

The title compound **26** was obtained in 69 % yield from compound **12** using compound **1** in a manner similar to that described for the synthesis of compound **21.** ¹H NMR (300 MHz, CDCl₃): 1.15 (d, J = 6.9 Hz, 6H), 3.26 (m, 1H), 3.94 (s, 3H), 4.88 (s, 2H), 4.99 (s, 2H), 5.10 (s, 2H), 6.35 (s, 1H), 6.75 (d, J = 7.5 Hz, 2H), 7.02 (d, J = 8.4 Hz, 2H), 7.11 (s, 1H), 7.35 - 7.44 (m, 12H), 7.86 (d, J = 8.4 Hz, 2H).

### Example 24 Synthesis of methyl 4-((2,4-bis(benzyloxy)-N-(4-iodophenyl)-5-isopropylbenzamido)methyl)benzoate (27)

The title compound **27** was obtained in 72 % yield from compound **13** using compound **1** in a manner similar to that described for the synthesis of compound **21.** ¹H NMR (300 MHz, CDCl₃): 1.15 (d, J = 6.9 Hz, 6H), 3.25 (m, 1H), 3.94 (s, 3H), 4.86 (s, 2H), 4.99 (s, 2H), 5.09 (s, 2H), 6.31 (s, 1H), 6.57 (d, J = 8.1 Hz, 2H), 7.11 (s, 1H), 7.30 - 7.40 (m, 14 H), 7.86 (d, J = 8.4 Hz, 2H).

### Example 25 Synthesis of methyl 4-((N-(benzo[d][1,3]dioxol-5-yl)-2,4-bis(benzyloxy)-5-isopropylbenzamido)methyl)benzoate (28)

The title compound **28** was obtained in 78 % yield from compound **14** using compound **1** in a manner similar to that described for the synthesis of compound **21.** ¹H NMR (300 MHz, CDCl₃): 0.92 (d, J = 6.9 Hz, 6H), 3.22 (m, 1H), 3.82 (s, 3H), 4.96 (s, 2H), 4.99 (s, 2H), 5.09 (s, 2H), 5.93 (s, 2H), 6.29 (s, 1H), 6.57 (dd, J = 2.1 and 8.1 Hz, 1H), 6.67 (d, J = 2.1 Hz, 1H), 6.76 (d, J = 8.4 Hz, 1H), 6.87 (s, 1H), 7.32 - 7.49 (m, 12H), 7.68 (d, J = 8.1 Hz, 2H).

### Example 26 Synthesis of methyl 4-((2,4-bis(benzyloxy)-5-isopropyl-N-(4-morpholinophenyl)benzamido)methyl)benzoate (29)

The title compound **29** was obtained in 83 % yield from compound **15** using compound **1** in a manner similar to that described for the synthesis of compound **21.** ¹H NMR (300 MHz, CDCl₃): 0.87 (d, J = 6.6 Hz, 6H), 2.91 (m, 1H), 3.16 (t, J = 4.8 Hz, 4H), 3.72 (t, J = 4.5 Hz, 4H), 3.93 (s, 3H), 4.99 (s, 2H), 5.09 (s, 4H), 6.41 (s, 1H), 6.71 (s, 1H), 6.89 (d, J = 9.0 Hz, 2H), 6.97 (d, J = 9.0 Hz, 2H), 7.31 - 7.40 (m, 12H), 7.79 (d, J = 8.1 Hz, 2H).

### Example 27 Synthesis of methyl 4-((2,4-bis(benzyloxy)-N-(4-(dimethylamino)phenyl)-5-isopropylbenzamido)methyl)benzoate (30)

The title compound **30** was obtained in 76 % yield from compound **16** using compound **1** in a manner similar to that described for the synthesis of compound **21.** ¹H NMR (300 MHz, CDCl₃) : 0.86 (d, J = 6.9 Hz, 6H), 2.97 (s, 3H), 2.99 (s, 3H), 3.11 (m, 1H), 3.81 (s, 3H), 4.99 (s, 4H), 5.13 (s, 2H), 6.22 (s, 1H), 6.64 - 6.69 (m, 3H), 6.89 (d, J = 6.9 Hz, 2H), 7.35 - 7.43 (m, 12H), 7.69 (d, J = 8.1 Hz, 2H).

### Example 28 Synthesis of methyl 4-((2,4-bis(benzyloxy)-5-isopropyl-N-(4-(4-methylpiperazin-1-yl)phenyl)benzamido)methyl)benzoate (31)

The title compound **31** was obtained in 78 % yield from compound **17** using compound **1** in a manner similar to that described for the synthesis of compound **21.** ¹H NMR (300 MHz, CDCl₃): 0.89 (d, J = 6.9 Hz, 6H), 2.32 (s, 3H), 2.68 (t, J = 5.1 Hz, 4H), 2.94 (m, 1H), 3.23 (t, J = 4.8 Hz, 4H), 3.83 (s, 3H), 4.99 (s, 2H), 5.12 (s, 4H), 6.24 (s, 1H), 6.71 (s, 1H), 6.83 (d, J = 8.7 Hz, 2H), 6.91 (d, J = 8.7 Hz, 2H), 7.35 - 7.42 (m, 12H), 7.69 (d, J = 7.5 Hz, 2H).

### Example 29 Synthesis of methyl 4-((2,4-bis(benzyloxy)-5-isopropyl-N-(4-(piperidin-1-yl)phenyl)benzamido)methyl)benzoate (32)

The title compound **32** was obtained in 72 % yield from compound **18** using compound **1** in a manner similar to that described for the synthesis of compound **21.** ¹H NMR (300 MHz, CDCl₃): 1.10 (d, J = 6.9 Hz, 6H), 1.57 - 1.67 (m, 6H), 3.07 (bs, 4H), 3.19 (m, 1H), 3.69 (s, 3H), 5.00 (s, 2H), 5.17 (s, 4H), 6.73-6.98 (m, 6H), 7.35 - 7.43 (m, 12H), 7.80 (d, J = 8.1 Hz, 2H).

### Example 30 Synthesis of methyl 4-((2,4-bis(benzyloxy)-5-isopropyl-N-(4-methoxyphenyl)benzamido)methyl)benzoate (33)

The title compound **33** was obtained in 75 % yield from compound **19** using compound **1** in a manner similar to that described for the synthesis of compound **21.** ¹H NMR (300 MHz, CDCl₃): 0.99 (d, J = 6.9 Hz, 6H), 2.98 (m, 1H), 3.76 (s, 3H), 3.81 (s, 3H), 4.99 (s, 4H), 5.16 (s, 2H), 6.21 (s, 1H), 6.75 (s, 1H), 6.89 (d, J = 9.0 Hz, 2H), 6.93 (d, J = 9.0 Hz, 2H), 7.27 - 7.43 (m, 12H), 7.74 (d, J = 8.1 Hz, 2H).

### Example 31 Synthesis of methyl 4-((2,4-bis(benzyloxy)-N-(4-(benzyloxy)phenyl)-5-isopropylbenzamido)methyl)benzoate (34)

The title compound **34** was obtained in 75 % yield from compound **20** using compound **1** in a manner similar to that described for the synthesis of compound **21.** ¹H NMR (300 MHz, CDCl₃): 0.98 (d, J = 6.9 Hz, 6H), 2.99 (m, 1H), 3.85 (s, 3H), 4.89 (s, 4H), 5.01 (s, 2H), 5.14 (s, 2H), 6.23 (s, 1H), 6.67 (d, J = 9.0 Hz, 2H), 6.71 (s, 1H), 6.88 (d, J = 8.7 Hz, 2H), 7.25 - 7.431 (m, 17H), 7.82 (d, J = 8.1 Hz, 2H).

### Example 32 Synthesis of 4-((2,4-bis(benzyloxy)-5-isopropyl-N-methylbenzamido)methyl)benzoic acid (35)

The title compound **35** was obtained in 92% yield from compound **21** in a manner similar to that described for the synthesis of compound **3.** ¹H NMR (300 MHz, CD₃OD): 1.11 (d, J = 6.9 Hz, 6H), 2.83 (s, 3H), 3.29 (m, 1H), 4.81 (s, 2H), 4.92 (s, 2H), 5.21 (s, 2H), 6.31 (s, 1H), 7.11 (s, 1H), 7.32 - 7.38 (m, 12H), 7.65 (d, J = 8.4 Hz, 2H).

### Example 33 Synthesis of 4-((2,4-bis(benzyloxy)-N-ethyl-5-isopropylbenzamido)methyl)benzoic acid (36)

The title compound **36** was obtained in 91% yield from compound **22** in a manner similar to that described for the synthesis of compound **3.** ¹H NMR (300 MHz, CD₃OD): 1.12 (d, J = 6.9 Hz, 6H), 1.27 (t, J = 5.4 Hz, 3H), 3.21 (m, 1H), 3.43 (q, J = 5.4 Hz, 2H), 4.73 (s, 2H), 5.11 (s, 4H), 6.51 (s, 1H), 6.93 (s, 1H), 7.37 - 7.42 (m, 12H), 7.84 (d, J = 8.1 Hz, 2H).

### Example 34 Synthesis of 4-((2,4-bis(benzyloxy)-5-isopropyl-N-propylbenzamido)methyl)benzoic acid (37)

The title compound **37** was obtained in 89% yield from compound **23** in a manner similar to that described for the synthesis of compound **3.** ¹H NMR (300 MHz, CD₃OD): 0.97 (t, J = 7.5 Hz, 3H), 1.11 (d, J = 6.6 Hz, 6H), 1.79 (m, 2H), 3.24 (m, 1H), 3.35 (t, J = 7.5 Hz, 2H), 4.89 (s, 2H), 5.17 (s, 4H), 6.39 (s, 1H), 6.97 (s, 1H), 7.38 - 7.45 (m, 12H), 7.68 (d, J = 8.1 Hz, 2H).

### Example 35 Synthesis of 4-((2,4-bis(benzyloxy)-5-isopropyl-N-phenylbenzamido)methyl)benzoic acid (38)

The title compound **38** was obtained in 95 % yield from compound **24** in a manner similar to that described for the synthesis of compound 3. ¹H NMR (300 MHz, CD₃OD): 0.98 (d, J = 6.9 Hz, 6H), 2.99 (m, 1H), 4.87 (s, 2H), 5.16 (s, 4H), 6.39 (s, 1H), 6.67 (s, 1H), 7.18 (d, J = 7.2 Hz, 2H), 7.23 (t, J = 6.6 Hz, 1H), 7.29 - 7.41 (m, 14H), 7.68 (d, J = 8.1 Hz, 2H).

### Example 36 Synthesis of 4-((2,4-bis(benzyloxy)-N-(4-fluorophenyl)-5-isopropylbenzamido)methyl)benzoic acid (39)

The title compound **39** was obtained in 93 % yield from compound **25** in a manner similar to that described for the synthesis of compound **3.** ¹H NMR (300 MHz, CD₃OD): 1.17 (d, J = 6.9 Hz, 6H), 3.14 (m, 1H), 4.92 (s, 2H), 5.16 (s, 4H) 6.37 (s, 1H), 6.75 - 6.81 (m, 4H), 7.12 (s, 1H), 7.36 - 7.41 (m, 12H), 7.82 (d, J = 8.1 Hz, 2H).

### Example 37 Synthesis of 4-((2,4-bis(benzyloxy)-N-(4-chlorophenyl)-5-isopropylbenzamido)methyl)benzoic acid (40)

The title compound **40** was obtained in 91 % yield from compound **26** in a manner similar to that described for the synthesis of compound **3.** ¹H NMR (300 MHz, CD₃OD): 1.11 (d, J = 6.9 Hz, 6H), 3.21 (m, 1H), 4.82 (s, 2H), 4.91 (s, 2H), 5.15 (s, 2H), 6.31 (s, 1H), 6.72 (d, J = 7.5 Hz, 2H), 7.09 (d, J = 8.4 Hz, 2H), 7.15 (s, 1H), 7.31 - 7.43 (m, 12H), 7.82 (d, J = 8.4 Hz, 2H).

### Example 38 Synthesis of 4-((2,4-bis(benzyloxy)-N-(4-iodophenyl)-5-isopropylbenzamido)methyl)benzoic acid (41)

The title compound **41** was obtained in 92 % yield from compound **27** in a manner similar to that described for the synthesis of compound **3.** ¹H NMR (300 MHz, CD₃OD): 1.18 (d, J = 6.9 Hz, 6H), 3.21 (m, 1H), 4.82 (s, 2H), 4.93 (s, 2H), 5.19 (s, 2H), 6.39 (s, 1H), 6.68 (d, J = 8.1 Hz, 2H), 7.15 (s, 1H), 7.27 - 7.45 (m, 14 H), 7.82 (d, J = 8.4 Hz, 2H).

### Example 39 Synthesis of 4-((N-(benzo[d][1,3]dioxol-5-yl)-2,4-bis(benzyloxy)-5-isopropylbenzamido)methyl)benzoic acid (42)

The title compound **42** was obtained in 91 % yield from compound **28** in a manner similar to that described for the synthesis of compound **3.** ¹H NMR (300 MHz, CD₃OD): 0.98 (d, J = 6.9 Hz, 6H), 3.18 (m, 1H), 4.99 (s, 4H), 5.08 (s, 2H), 5.98 (s, 2H), 6.32 (s, 1H), 6.52 (dd, J = 2.1 and 8.1 Hz, 1H), 6.62 (d, J = 2.1 Hz, 1H), 6.79 (d, J = 8.4 Hz, 1H), 6.92 (s, 1H), 7.26 - 7.42 (m, 12H), 7.62 (d, J = 8.1 Hz, 2H).

### Example 40 Synthesis of 4-((2,4-bis(benzyloxy)-5-isopropyl-N-(4-morpholinophenyl)benzamido)methyl)benzoic acid (43)

The title compound **43** was obtained in 88 % yield from compound **29** in a manner similar to that described for the synthesis of compound **3.** ¹H NMR (300 MHz, CD₃OD): 0.91 (d, J = 6.6 Hz, 6H), 2.98 (m, 1H), 3.15 (t, J = 4.8 Hz, 4H), 3.87 (t, J = 4.5 Hz, 4H), 5.01 (s, 2H), 5.08 (s, 4H), 6.49 (s, 1H), 6.74 (s, 1H), 6.92 (d, J = 9.0 Hz, 2H), 6.99 (d, J = 9.0 Hz, 2H), 7.35 - 7.43 (m, 12H), 7.81 (d, J = 8.1 Hz, 2H).

### Example 41 Synthesis of 4-((2,4-bis(benzyloxy)-N-(4-(dimethylamino)phenyl)-5-isopropylbenzamido)methyl)benzoic acid (44)

The title compound **44** was obtained in 81 % yield from compound **30** in a manner similar to that described for the synthesis of compound **3.** ¹H NMR (300 MHz, CD₃OD): 0.96 (d, J = 6.6 Hz, 6H), 2.99 (s, 3H), 3.02 (s, 3H), 3.18 (m, 1H), 5.03 (s, 4H), 5.18 (s, 2H), 6.31 (s, 1H), 6.62 - 6.67 (m, 3H), 6.86 (d, J = 7.2 Hz, 2H), 7.32 - 7.41 (m, 12H), 7.62 (d, J = 8.1 Hz, 2H).

### Example 42 Synthesis of 4-((2,4-bis(benzyloxy)-5-isopropyl-N-(4-(4-methylpiperazin-1-yl)phenyl)benzamido)methyl)benzoic acid (45)

The title compound **45** was obtained in 87 % yield from compound **31** in a manner similar to that described for the synthesis of compound **3.** ¹H NMR (300 MHz, CD₃OD): 0.98 (d, J = 6.6 Hz, 6H), 2.38 (s, 3H), 2.75 (t, J = 5.1 Hz, 4H), 2.98 (m, 1H), 3.34 (t, J = 4.8 Hz, 4H), 4.92 (s, 2H), 5.14 (s, 4H), 6.32 (s, 1H), 6.68 (s, 1H), 6.87 (d, J = 8.1 Hz, 2H), 6.96 (d, J = 8.1 Hz, 2H), 7.32 - 7.48 (m, 12H), 7.78 (d, J = 7.5 Hz, 2H).

### Example 43 Synthesis of 4-((2,4-bis(benzyloxy)-5-isopropyl-N-(4-(piperidin-1-yl)phenyl)benzamido)methyl)benzoic acid (46)

The title compound **46** was obtained in 83 % yield from compound **32** in a manner similar to that described for the synthesis of compound **3.** ¹H NMR (300 MHz, CD₃OD): 0.89 (d, J = 6.6 Hz, 6H), 1.55 - 1.72 (m, 6H), 3.23 (m, 1H), 3.34 (t, J = 4.5 Hz, 4H), 4.98 (s, 2H), 5.13 (s, 4H), 6.39 (s, 1H), 6.45 (s, 1H), 6.97 (d, J = 8.7 Hz, 2H), 7.01 (d, J = 8.1 Hz, 2H), 7.35 - 7.38 (m, 12H), 7.72 (d, J = 7.2 Hz, 2H).

### Example 44 Synthesis of 4-((2,4-bis(benzyloxy)-5-isopropyl-N-(4-methoxyphenyl)benzamido)methyl)benzoic acid (47)

The title compound **47** was obtained in 89 % yield from compound **33** in a manner similar to that described for the synthesis of compound **3.** ¹H NMR (300 MHz, CD₃OD): 0.93 (d, J = 6.6 Hz, 6H), 2.97 (m, 1H), 3.85 (s, 3H), 4.93 (s, 4H), 5.18 (s, 2H), 6.27 (s, 1H), 6.78 (s, 1H), 6.82 (d, J = 8.4 Hz, 2H), 6.95 (d, J = 8.1 Hz, 2H), 7.21 - 7.33 (m, 12H), 7.68 (d, J = 8.1 Hz, 2H).

### Example 45 Synthesis of 4-((2,4-bis(benzyloxy)-N-(4-(benzyloxy)phenyl)-5-isopropylbenzamido)methyl)benzoic acid (48)

The title compound **48** was obtained in 94 % yield from compound **34** in a manner similar to that described for the synthesis of compound 3. ¹H NMR (300 MHz, CDCl₃): 0.92 (d, J = 6.6 Hz, 6H), 2.92 (m, 1H), 4.99 (s, 4H), 5.11 (s, 2H), 5.17 (s, 2H), 6.31 (s, 1H), 6.62 (d, J = 8.1 Hz, 2H), 6.78 (s, 1H), 6.98 (d, J = 8.4 Hz, 2H), 7.29 - 7.45 (m, 17H), 7.78 (d, J = 8.4 Hz, 2H).

### Example 46 Synthesis of 2,4-bis(benzyloxy)-N-(4-((benzyloxy)carbamoyl)benzyl)-5-isopropyl-N-methylbenzamide (49)

The title compound **49** was obtained in 71 % yield from compound **35** using NH₂OBn. HCl in a manner similar to that described for the synthesis of compound **4.** ¹H NMR (300 MHz, CD₃OD): 1.13 (d, J = 6.6 Hz, 6H), 2.89 (s, 3H), 3.19 (m, 1H), 4.89 (s, 2H), 4.96 (s, 2H), 5.23 (s, 4H), 6.37 (s, 1H), 7.16 (s, 1H), 7.31 - 7.35 (m, 17H), 7.61 (d, J = 8.1 Hz, 2H).

### Example 47 Synthesis of 2,4-bis(benzyloxy)-N-(4-((benzyloxy)carbamoyl)benzyl)-N-ethyl-5-isopropylbenzamide (50)

The title compound **50** was obtained in 68 % yield from compound **36** using NH₂OBn. HCl in a manner similar to that described for the synthesis of compound **4.** ¹H NMR (300 MHz, CD₃OD): 1.15 (d, J = 6.9 Hz, 6H), 1.21 (t, J = 5.4 Hz, 3H), 3.19 (m, 1H), 3.32 (q, J = 5.4 Hz, 2H), 4.84 (s, 2H), 5.11 (s, 4H), 5.21 (s, 2H), 6.47 (s, 1H), 6.99 (s, 1H), 7.32 - 7.41 (m, 17H), 7.82 (d, J = 8.4 Hz, 2H).

### Example 48 Synthesis of 2,4-bis(benzyloxy)-N-(4-((benzyloxy)carbamoyl)benzyl)-5-isopropyl-N-propylbenzamide (51)

The title compound **51** was obtained in 73 % yield from compound **37** using NH₂OBn. HCl in a manner similar to that described for the synthesis of compound **4.** ¹H NMR (300 MHz, CD₃OD): 0.92 (t, J = 7.2 Hz, 3H), 1.19 (d, J = 6.9 Hz, 6H), 1.83 (m, 2H), 3.17 (m, 1H), 3.31 (t, J = 7.2 Hz, 2H), 4.89 (s, 2H), 5.17 (s, 4H), 5.21 (s, 2H), 6.45 (s, 1H), 6.92 (s, 1H), 7.33 - 7.49 (m, 17H), 7.63 (d, J = 8.1 Hz, 2H).

### Example 49 Synthesis of 2,4-bis(benzyloxy)-N-(4-((benzyloxy)carbamoyl)benzyl)-5-isopropyl-N-phenylbenzamide (52)

The title compound **52** was obtained in 75 % yield from compound **38** using NH₂OBn. HCl in a manner similar to that described for the synthesis of compound **4.** ¹H NMR (300 MHz, CD₃OD): 1.18 (d, J = 6.6 Hz, 6H), 2.98 (m, 1H), 4.87 (s, 2H), 5.16 (s, 6H), 6.38 (s, 1H), 6.65 (s, 1H), 7.16 (d, J = 7.2 Hz, 2H), 7.22 (t, J = 6.6 Hz, 1H), 7.35 - 7.41 (m, 19H), 7.69 (d, J = 8.4 Hz, 2H).

### Example 50 Synthesis of 2,4-bis(benzyloxy)-N-(4-((benzyloxy)carbamoyl)benzyl)-N-(4-fluorophenyl)-5-isopropylbenzamide (53)

The title compound **53** was obtained in 71 % yield from compound **39** using NH₂OBn. HCl in a manner similar to that described for the synthesis of compound **4.** ¹H NMR (300 MHz, CD₃OD): 1.15 (d, J = 6.6 Hz, 6H), 3.19 (m, 1H), 4.92 (s, 2H), 5.16 (s, 4H), 5.21 (s, 2H), 6.32 (s, 1H), 6.69 - 6.79 (m, 4H), 7.17 (s, 1H), 7.36 - 7.41 (m, 17H), 7.80 (d, J = 8.4 Hz, 2H).

### Example 51 Synthesis of 2,4-bis(benzyloxy)-N-(4-((benzyloxy)carbamoyl)benzyl)-N-(4-chlorophenyl)-5-isopropylbenzamide (54)

The title compound **54** was obtained in 76 % yield from compound **40** using NH₂OBn. HCl in a manner similar to that described for the synthesis of compound **4.** ¹H NMR (300 MHz, CD₃OD): 1.14 (d, J = 6.6 Hz, 6H), 3.25 (m, 1H), 4.82 (s, 2H), 4.91 (s, 2H), 5.15 (s, 4H), 6.38 (s, 1H), 6.78 (d, J = 7.2 Hz, 2H), 7.19 (d, J = 8.1 Hz, 2H), 7.25 (s, 1H), 7.34 - 7.45 (m, 17H), 7.78 (d, J = 8.1 Hz, 2H).

### Example 52 Synthesis of 2,4-bis(benzyloxy)-N-(4-((benzyloxy)carbamoyl)benzyl)-N-(4-iodophenyl)-5-isopropylbenzamide (55)

The title compound **55** was obtained in 69 % yield from compound **41** using NH₂OBn. HCl in a manner similar to that described for the synthesis of compound **4.** ¹H NMR (300 MHz, CD₃OD): 1.13 (d, J = 6.6 Hz, 6H), 3.18 (m, 1H), 4.82 (s, 2H), 4.93 (s, 2H), 5.19 (s, 4H), 6.32 (s, 1H), 6.81 (d, J = 8.4 Hz, 2H), 7.19 (s, 1H), 7.29 - 7.42 (m, 19 H), 7.82 (d, J = 8.1 Hz, 2H).

### Example 53 Synthesis of N-(benzo[d][1,3]dioxol-5-yl)-2,4-bis(benzyloxy)-N-(4-((benzyloxy)carbamoyl)benzyl)-5-isopropylbenzamide (56)

The title compound **56** was obtained in 79 % yield from compound **42** using NH₂OBn. HCl in a manner similar to that described for the synthesis of compound **4.** ¹H NMR (300 MHz, CD₃OD) : 1.18 (d, J = 6.9 Hz, 6H), 3.23 (m, 1H), 4.99 (s, 4H), 5.08 (s, 4H), 5.99 (s, 2H), 6.31 (s, 1H), 6.58 (dd, J = 2.4 and 8.4 Hz, 1H), 6.69 (d, J = 2.4 Hz, 1H), 6.92 (d, J = 8.4 Hz, 1H), 6.99 (s, 1H), 7.21 - 7.48 (m, 17H), 7.68 (d, J = 8.4 Hz, 2H).

### Example 54 Synthesis of 2,4-bis(benzyloxy)-N-(4-((benzyloxy)carbamoyl)benzyl)-5-isopropyl-N-(4-morpholinophenyl)benzamide (57)

The title compound **57** was obtained in 72 % yield from compound **43** using NH₂OBn. HCl in a manner similar to that described for the synthesis of compound **4.** ¹H NMR (300 MHz, CD₃OD): 0.98 (d, J = 6.6 Hz, 6H), 2.91 (m, 1H), 3.19 (t, J = 4.8 Hz, 4H), 3.89 (t, J = 4.8 Hz, 4H), 5.04 (s, 4H), 5.08 (s, 4H), 6.56 (s, 1H), 6.78 (s, 1H), 6.99 (d, J = 8.7 Hz, 2H), 7.8 (d, J = 8.7 Hz, 2H), 7.32 - 7.41 (m, 17H), 7.87 (d, J = 8.4 Hz, 2H).

### Example 55 Synthesis of 2,4-bis(benzyloxy)-N-(4-((benzyloxy)carbamoyl)benzyl)-N-(4-(dimethylamino)phenyl)-5-isopropylbenzamide (58)

The title compound **58** was obtained in 62 % yield from compound **44** using NH₂OBn. HCl in a manner similar to that described for the synthesis of compound **4.** ¹H NMR (300 MHz, CD₃OD): 0.98 (d, J = 6.9 Hz, 6H), 3.12 (s, 3H), 3.22 (s, 3H), 3.28 (m, 1H), 5.03 (s, 4H), 5.18 (s, 4H), 6.37 (s, 1H), 6.57 - 6.65 (m, 3H), 6.89 (d, J = 7.8 Hz, 2H), 7.35 - 7.45 (m, 17H), 7.64 (d, J = 8.4 Hz, 2H).

### Example 56 Synthesis of 2,4-bis(benzyloxy)-N-(4-((benzyloxy)carbamoyl)benzyl)-5-isopropyl-N-(4-(4-methylpiperazin-1-yl)phenyl)benzamide (59)

The title compound **59** was obtained in 65 % yield from compound **45** using NH₂OBn. HCl in a manner similar to that described for the synthesis of compound **4.** ¹H NMR (300 MHz, CD₃OD): 1.12 (d, J = 6.9 Hz, 6H), 2.41 (s, 3H), 2.79 (t, J = 5.1 Hz, 4H), 2.93 (m, 1H), 3.38 (t, J = 4.8 Hz, 4H), 4.99 (s, 2H), 5.02 (s, 2H), 5.14 (s, 4H), 6.38 (s, 1H), 6.79 (s, 1H), 6.89 (d, J = 8.4 Hz, 2H), 6.98 (d, J = 8.4 Hz, 2H), 7.32 - 7.42 (m, 17H), 7.72 (d, J = 7.8 Hz, 2H).

### Example 57 Synthesis of 2,4-bis(benzyloxy)-N-(4-((benzyloxy)carbamoyl)benzyl)-5-isopropyl-N-(4-(piperidin-1-yl)phenyl)benzamide (60)

The title compound **60** was obtained in 65 % yield from compound **46** using NH₂OBn. HCl in a manner similar to that described for the synthesis of compound **4.** ¹H NMR (300 MHz, CD₃OD): 0.97 (d, J = 6.9 Hz, 6H), 1.58 - 1.71 (m, 6H), 3.13 (m, 1H), 3.23 (t, J = 4.8 Hz, 4H), 4.97 (s, 2H), 5.07 (s, 2H), 5.13 (s, 4H), 6.42 (s, 1H), 6.48 (s, 1H), 6.99 (d, J = 8.1 Hz, 2H), 7.11 (d, J = 8.4 Hz, 2H), 7.32 - 7.39 (m, 17H), 7.71 (d, J = 7.8 Hz, 2H).

### Example 58 Synthesis of 2,4-bis(benzyloxy)-N-(4-((benzyloxy)carbamoyl)benzyl)-5-isopropyl-N-(4-methoxyphenyl)benzamide (61)

The title compound **61** was obtained in 69 % yield from compound **47** using NH₂OBn. HCl in a manner similar to that described for the synthesis of compound **4.** ¹H NMR (300 MHz, CD₃OD): 0.98 (d, J = 6.9 Hz, 6H), 2.91 (m, 1H), 3.82 (s, 3H), 4.99 (s, 4H), 5.18 (s, 4H), 6.37 (s, 1H), 6.79 (s, 1H), 6.81 (d, J = 8.1 Hz, 2H), 6.99 (d, J = 8.4 Hz, 2H), 7.25 - 7.38 (m, 17H), 7.71 (d, J = 8.4 Hz, 2H).

### Example 59 Synthesis of 2,4-bis(benzyloxy)-N-(4-((benzyloxy)carbamoyl)benzyl)-N-(4-(benzyloxy)phenyl)-5-isopropylbenzamide (62)

The title compound **62** was obtained in 71 % yield from compound **48** using NH₂OBn. HCl in a manner similar to that described for the synthesis of compound **4.** ¹H NMR (300 MHz, CDCl₃) : 0.98 (d, J = 6.9 Hz, 6H), 2.98 (m, 1H), 4.99 (s, 4H), 5.11 (s, 4H), 5.17 (s, 2H), 6.39 (s, 1H), 6.58 (d, J = 8.4 Hz, 2H), 6.76 (s, 1H), 7.01 (d, J = 8.1 Hz, 2H), 7.32 - 7.41 (m, 22H), 7.69 (d, J = 8.1 Hz, 2H).

### Example 60 Synthesis of 2,4-dihydroxy-N-(4-(hydroxycarbamoyl)benzyl)-5-isopropyl-N-methylbenzamide (63)

Compound **49** (200 mg, 0.31 mmol) was dissolved in CH₃OH (10 mL). A catalytic amount of 10% palladium on carbon was added and the reaction mixture was stirred for 4 hours under hydrogen. The reaction mixture was filtered over celite and the filtrate was dried in vacuum to obtain a residue that was purified by silica gel chromatography (EtOAc:CH3OH: 9.5:0.5) to give compound **63** in 82 % yield; ¹H NMR (300 MHz, CD₃OD): 1.15 (d, J = 6.9 Hz, 6H), 2.99 (s, 3H), 3.20 (m, 1H), 4.75 (s, 2H), 6.37 (s, 1H), 7.01 (s, 1H), 7.45 (d, J = 8.1 Hz, 2H), 7.77 (d, J = 8.4 Hz, 2H). HRMS (ESI) for C₁₉H₂₃N₂O₅ (M+H⁺): calcd, 359.1607; found, 359.1608.

### Example 61 Synthesis of N-ethyl-2,4-dihydroxy-N-(4-(hydroxycarbamoyl)benzyl)-5-isopropylbenzamide (64)

The title compound **64** was synthesized in 86 % yield from compound **50** in a manner similar to that described for compound **63.** ¹H NMR (300 MHz, CD₃OD): 1.13 (d, J = 6.3 Hz, 6H), 1.20 (t, J = 5.4 Hz, 3H), 3.14 (m, 1H), 3.34 (q, J = 5.4 Hz, 2H), 4.74 (s, 2H), 6.41 (s, 1H), 6.97 (s, 1H), 7.45 (d, J = 7.8 Hz, 2H), 7.76 (d, J = 8.1 Hz, 2H). HRMS (ESI) for C₂₀H₂₅N₂O₅ (M+H⁺): calcd, 373.1763; found, 373.1765.

### Example 62 Synthesis of 2,4-dihydroxy-N-(4-(hydroxycarbamoyl)benzyl)-5-isopropyl-N-propylbenzamide (65)

The title compound **65** was synthesized in 84 % yield from compound **51** in an manner similar to that described for compound **63.** ¹H NMR (300 MHz, CD₃OD): 0.89 (t, J = 7.5 Hz, 3H), 1.14 (d, J = 6.6 Hz, 6H), 1.59 (m, 2H), 3.17 (m, 1H), 3.34 (t, J = 7.5 Hz, 2H), 4.75 (s, 2H), 6.39 (s, 1H), 6.96 (s, 1H), 7.44 (d, J = 7.8 Hz, 2H), 7.75 (d, J = 8.1 Hz, 2H). HRMS (ESI) for C₂₁H₂₇N₂O₅ (M+H⁺): calcd, 387.1920; found, 387.1922.

### Example 63 Synthesis of 2,4-dihydroxy-N-(4-(hydroxycarbamoyl)benzyl)-5-isopropyl-N-phenylbenzamide (66)

The title compound **66** was synthesized in 79 % yield from compound **52** in a manner similar to that described for compound **63.** ¹H NMR (300 MHz, CD₃OD): 0.81 (d, J = 6.6 Hz, 6H), 2.92 (m, 1H), 5.20 (s, 2H), 6.23 (s, 1H), 6.69 (s, 1H), 7.10 (d, J = 7.2 Hz, 2H), 7.22 (t, J = 6.6 Hz, 1H), 7.24 - 7.31 (m, 2H), 7.44 (d, J = 8.1 Hz, 2H), 7.70 (d, J = 8.1 Hz, 2H). HRMS (ESI) for C₂₄H₂₅N₂O₅ (M+H⁺): calcd, 421.1763; found, 421.1761.

### Example 64 Synthesis of N-(4-fluorophenyl)-2,4-dihydroxy-N-(4-(hydroxycarbamoyl)benzyl)-5-isopropylbenzamide (67)

Compound **53** (100 mg, 0.14 mmol) was dissolved in DCM and BCl₃ (1 M in heptane, 6 eq) was added to the solution at 0 °C. The reaction mixture was stirred for 45 min at the same temperature. The reaction mixture was filtered to collect the solid precipitated solid (compound **67,** Yield - 64 %). ¹H NMR (300 MHz, CD₃OD): 0.88 (t, J = 6.6 Hz, 3H), 2.98 (m, 1H), 5.18 (s, 2H), 6.22 (s, 1H), 6.70 (s, 1H), 6.99 (t, J = 8.4 Hz, 2H), 7.10 (m, 2H), 7.44 (d, J = 8.1 Hz, 2H), 7.69 (d, J = 7.8 Hz, 2H). HRMS (ESI) for C₂₄H₂₅FN₂O₅ (M+H⁺): calcd, 439.1669; found, 439.1672.

### Example 65 Synthesis of N-(4-chlorophenyl)-2,4-dihydroxy-N-(4-(hydroxycarbamoyl)benzyl)-5-isopropylbenzamide (68)

The title compound **68** was synthesized in 71 % yield from compound **54** in a manner similar to that described for compound **67.** ¹H NMR (300 MHz, CD₃OD): 0.88 (d, J = 6.6 Hz, 3H), 2.98 (m, 1H), 5.19 (s, 2H), 6.23 (s, 1H), 6.70 (s, 1H), 7.07 (d, J = 8.7 Hz, 2H), 7.25 (d, J = 8.7 Hz, 2H), 7.44 (d, J = 8.1 Hz, 2H), 7.70 (d, J = 8.1 Hz, 2H). HRMS (ESI) for C₂₄H₂₄ClN₂O₅ (M+H⁺): calcd, 455.1374; found, 455.1377.

### Example 66 Synthesis of 2,4-dihydroxy-N-(4-(hydroxycarbamoyl)benzyl)-N-(4-iodophenyl)-5-isopropylbenzamide (69)

The title compound **69** was synthesized in 73 % yield from compound **55** in a manner similar to that described for compound **67.** ¹H NMR (300 MHz, CD₃OD): 0.87 (d, J = 6.6 Hz, 6H), 2.99 (m, 1H), 5.17 (s, 2H), 6.25 (s, 1H), 6.67 (s, 1H), 6.87 (d, J = 8.7 Hz, 2H), 7.42 (d, J = 8.1 Hz, 2H), 7.58 (d, J = 8.4 Hz, 2H), 7.69 (d, J = 7.8 Hz, 2H). HRMS (ESI) for C₂₄H₂₄ClN₂O₅ (M+H⁺): calcd, 547.0730; found, 547.0735.

### Example 67 Synthesis of N-(benzo[d][1,3]dioxol-5-yl)-2,4-dihydroxy-N-(4-(hydroxycarbamoyl)benzyl)-5-isopropylbenzamide (70)

The title compound **70** was synthesized in 81 % yield from compound **56** in a manner similar to that described for compound **63.** ¹H NMR (300 MHz, CD₃OD): 0.90 (s, 6H), 3.02 (m, 1H), 5.14 (s, 2H), 5.92 (s, 2H), 6.24 (s, 1H), 6.54 (dd, J = 2.1 and 8.1 Hz, 1H), 6.61 (d, J = 2.1 Hz, 1H), 6.69 (d, J = 8.4 Hz, 1H), 6.77 (s, 1H), 7.44 (d, J = 8.4 Hz, 2H), 7.71 (d, J = 8.1 Hz, 2H). HRMS (ESI) for C₂₅H₂₅N₂O₇ (M+H⁺): calcd, 465.1662; found, 465.1661.

### Example 68 Synthesis of 2,4-dihydroxy-N-(4-(hydroxycarbamoyl)benzyl)-5-isopropyl-N-(4-morpholinophenyl)benzamide (71)

The title compound **71** was synthesized in 79 % yield from compound **57** in a manner similar to that described for compound **63.** ¹H NMR (300 MHz, CD₃OD): 0.81 (d, J = 6.6 Hz, 6H), 2.94 (m, 1H), 3.06 (t, J = 4.8 Hz, 4H), 3.78 (t, J = 4.5 Hz, 4H), 5.11 (s, 2H), 6.25 (s, 1H), 6.68 (s, 1H), 6.82 (d, J = 9.0 Hz, 2H), 6.95 (d, J = 9.0 Hz, 2H), 7.40 (d, J = 8.1 Hz, 2H), 7.69 (d, J = 8.1 Hz, 2H). HRMS (ESI) for C₂₈H₃₂N₃O₆ (M+H⁺): calcd, 506.2291; found, 506.2292.

### Example 69 Synthesis of N-(4-(dimethylamino)phenyl)-2,4-dihydroxy-N-(4-(hydroxycarbamoyl)benzyl)-5-isopropylbenzamide (72)

The title compound **72** was synthesized in 79 % yield from compound **58** in a manner similar to that described for compound **63.** ¹H NMR (300 MHz, CD₃OD): 0.81 (d, J = 6.9 Hz, 6H), 2.94 (m, 1H), 2.89 (s, 3H), 2.90 (s, 3H), 5.13 (s, 2H), 6.22 (d, J = 2.7 Hz, 1H), 6.64 - 6.69 (m, 3H), 6.89 (d, J = 6.9 Hz, 2H), 7.43 (d, J = 7.5 Hz, 2H), 7.69 (d, J = 6.3 Hz, 2H). HRMS (ESI) for C₂₆H₃₀N₃O₅ (M+H⁺): calcd, 464.2185; found, 464.2187.

### Example 70 Synthesis of 2,4-dihydroxy-N-(4-(hydroxycarbamoyl)benzyl)-5-isopropyl-N-(4-(4-methylpiperazin-1-yl)phenyl)benzamide (73)

The title compound **74** was synthesized in 69 % yield from compound **59** in a manner similar to that described for compound **63.** ¹H NMR (300 MHz, CD₃OD): 0.82 (d, J = 6.9 Hz, 6H), 2.37 (s, 3H), 2.62 (t, J = 5.1 Hz, 4H), 2.94 (m, 1H), 3.18 (t, J = 4.8 Hz, 4H), 5.14 (s, 2H), 6.22 (s, 1H), 6.69 (s, 1H), 6.87 (d, J = 8.7 Hz, 2H), 6.95 (d, J = 9.0 Hz, 2H), 7.43 (d, J = 8.1 Hz, 2H), 7.69 (d, J = 7.8 Hz, 2H). HRMS (ESI) for C₂₉H₃₅N₄O₅ (M+H⁺): calcd, 519.2607; found, 519.2614.

### Example 71 Synthesis of 2,4-dihydroxy-N-(4-(hydroxycarbamoyl)benzyl)-5-isopropyl-N-(4-(piperidin-1-yl)phenyl)benzamide (74)

The title compound **73** was synthesized in 79 % yield from compound **60** in a manner similar to that described for compound **63.** ¹H NMR (300 MHz, CD₃OD): 0.82 (d, J = 6.6 Hz, 6H), 1.57-1.66 (m, 6H), 2.91 (m, 1H), 3.10 (t, J = 4.5 Hz, 4H), 5.12 (s, 2H), 6.24 (s, 1H), 6.71 (s, 1H), 6.83 (d, J = 8.7 Hz, 2H), 6.91 (d, J = 8.7 Hz, 2H), 7.42 (d, J = 7.8 Hz, 2H), 7.69 (d, J = 7.5 Hz, 2H). HRMS (ESI) for C₂₉H₃₄N₃O₅ (M+H⁺): calcd, 504.2498; found, 504.2526.

### Example 72 Synthesis of 2,4-Dihydroxy-N-(4-hydroxycarbamoyl-benzyl)-5-isopropyl-N-(4-methoxy-phenyl)-benzamide (75)

The title compound **75** was synthesized in 83 % yield from compound **61** in a manner similar to that described for compound **63.** ¹H NMR (300 MHz, CD₃OD): 0.84 (d, J = 6.9 Hz, 6H), 2.97 (m, 1H), 3.74 (s, 3H), 5.15 (s, 2H), 6.22 (s, 1H), 6.68 (s, 1H), 6.82 (d, J = 9.0 Hz, 2H), 6.99 (d, J = 9.0 Hz, 2H), 7.43 (d, J = 8.1 Hz, 2H), 7.70 (d, J = 8.1 Hz, 2H). HRMS (ESI) for C₂₅H₂₇N₂O₆ (M+H⁺): calcd, 451.1869; found, 451.1873.

### Example 73 Synthesis of 2,4-Dihydroxy-N-(4-hydroxycarbamoyl-benzyl)-N-(4-hydroxy-phenyl)-5-isopropyl-benzamide (76)

Compound **62** (300 mg, 0.366 mmol) was dissolved in DCM (20 mL) and BCl₃ (1M inhexane, 5 M1.0) was added to the solution at 0°C. The reaction mixture was stirred at room temperature for 2 h and quenched with water. Ethyl acetate (3 x 20 ml) was used for extraction. The combined organic layer was dried over anhydrous MgSO₄ and concentrated under reduced pressure. The residue was purified by silica gel chromatography (EtOAc) to give compound **76** in 78 % yield. ¹H NMR (300 MHz, CD₃OD): 0.86 (d, J = 6.9 Hz, 6H), 2.95 (m, 1H), 5.14 (s, 2H), 6.23 (s, 1H), 6.67 (d, J = 9.0 Hz, 2H), 6.71 (s, 1H), 6.88 (d, J = 8.7 Hz, 2H), 7.43 (d, J = 8.4 Hz, 2H), 7.82 (d, J = 8.1 Hz, 2H). HRMS (ESI) for C₂₄H₂₅N₂O₆ (M+H⁺): calcd, 437.1713; found, 437.1714.

### Biological Assay Examples

### Example 74 Growth inhibition of human colorectal cancer cells by HSP90/HDAC dual inhibitors

The inhibitory effect of the HSP90/HDAC6 dual inhibitors as described in Synthesis Examples on the survival rate of human colorectal cancer cells HCT116 is evaluated using MTT assay. The results show that the HSP90/HDAC6 dual inhibitors are particularly effective in inhibiting the growth of HCT116 cells as shown in Table 1.

**Table 1. HSP90/HDAC dual inhibitors inhibiting the survival of human colorectal cancer cells.**

| Compound | Human cell lines |
|---|---|
| | HCT116 |
| | IC50(µM±SD) |
| 63 | 1.09±0.08 |
| 64 | 3.69±0.09 |
| 66 | 0.90±0.09 |
| 67 | 4.61±0.11 |
| 68 | 5.28±0.92 |
| 69 | 1.16±0.01 |
| 70 | 0.25±0.04 |
| 71 | 0.47±0.02 |
| 72 | 0.90±0.18 |
| 73 | 1.25±0.13 |
| 74 | 4.83±0.47 |
| 75 | 0.27±0.01 |
| 76 | 0.83±0.01 |
| B055 | 0.5±0.08 |
| B056 | 2.45±0.28 |

### Example 75 Inhibitory ability of HSP90/HDAC dual inhibitors on HDAC6 activity

HSP90/HDAC dual inhibitors are evaluated for their ability to inhibit the activity of different subtypes of HDAC. The results are shown in Table 2, and they indicate that compounds 63 to 76 have the most significant effect on HDAC6 inhibition.

**Table 2. Inhibition of HDAC activity by HSP90/HDAC dual inhibitors for different subtypes of HDAC**

| Compound | HDAC1 | HDAC3 | HDAC6 |
|---|---|---|---|
| | IC50 (nM) | | |
| 63 | 1760.00 | 3620.00 | 5.93 |
| 64 | 5230.00 | >10000 | 23.00 |
| 66 | 3680.00 | 4740.00 | 23.90 |
| 67 | 1690.00 | 3140.00 | 22.90 |
| 68 | 1810.00 | 3030.00 | 17.20 |
| 69 | 1290.00 | 2170.00 | 9.17 |
| 70 | 1120.00 | 2200.00 | 12.90 |
| 71 | 275.00 | 691.00 | 16.00 |
| 72 | 1070.00 | 2190.00 | 13.70 |
| 73 | 173.00 | 645.00 | 5.88 |
| 74 | 1230.00 | 2700.00 | 70.50 |
| 75 | 616.00 | 1370.00 | 4.56 |
| 76 | 961.00 | 2840.00 | 6.54 |
| Trichostatin A | 10.50 | 24.50 | 3.34 |

| | | | |
|---|---|---|---|
| * Positive control: Trichostatin A | | | |

### Example 76 Inhibitory ability of HSP90/HDAC dual inhibitors on HSP90α

The HSP90/HDAC dual inhibitors are evaluated for their ability to inhibit the activity of HSP90α and HSP90β. As shown in Table 3, compounds 63 to 76 have the ability to inhibit the activity of HSP90α and HSP90β. Combining the above results (Table 2 and Table 3), compounds 63 to 76 have the ability to inhibit the activity of DHAC6 and HSP90.

**Table 3. Inhibition of HSP90α and HSP90β activities by HSP90/HDAC dual inhibitors**

| Compound | HSP90α | HSP90β |
|---|---|---|
| | IC₅₀ (nM) | |
| 63 | 25.20 | 38.80 |
| 64 | 93.90 | 190 |
| 66 | 54.50 | 200.00 |
| 67 | 40.80 | 103.00 |
| 68 | 59.90 | 159.00 |
| 69 | 91.40 | 272.00 |
| 70 | 39.90 | 95.60 |
| 71 | 16.30 | 18.70 |
| 72 | 32.20 | 117.00 |
| 73 | 36.70 | 60.50 |
| 74 | 98.70 | 373.00 |
| 75 | 52 | 66.9 |
| 76 | 35.00 | 51.80 |
| B055 | 15.3 | |
| B056 | 11.5 | |
| Geldanamycin | 22.40 | 15.60 |

| | | |
|---|---|---|
| *Positive control: Geldanamycin | | |

### Example 77 HSP90/HDAC dual inhibitors reduce IFN-γ-induced PD-L1 expression in colorectal cancer cells

Activated T cells and NK cells secrete IFN-γ to inhibit tumor growth. IFN-γ also upregulates PD-L1 expression on the tumor surface, which interacts with PD-1 on the T cell membrane, resulting in suppression of the cytotoxicity T cells to cancer cells and promoting immune escape of tumor cells. We evaluated whether the HSP90/HDAC dual inhibitors could downregulate the PD-L1 performance of IFN-γ-induced cancer cells. In flow cytometry analysis, IFN-γ was used to induce PD-L1 expression in HCT116 colorectal cancer cells, and the cells were further treated with the HSP90/HDAC dual inhibitors for 48 hours. The results show that the HSP90/HDAC dual inhibitors are effective in reducing the PD-L1 expression of IFN-γ-induced colorectal cancer cells (FIG. 1).

### Example 78 HSP90/HDAC dual inhibitor, Compound 75, inhibits the enhanced acetylation of HDAC6 and downregulates HSP90-related client proteins

We evaluated the ability of Compound 75 on the acetylation of α-tubulin and histone H3. HCT116 cells were administered with Compound 75 for 6 hours and analyzed by western blot. The results show that Compound 75 enhances the acetylation of α-tubulin and histone H3 (FIG. 2A). Next, we evaluated whether Compound 75 inhibited HSP90. Inhibition of HSP90 surrogately increases the expression of HSP70 and affects client proteins (e.g., Src, AKT, Rb, and FAK). HCT116 was administered with Compound75 for 48 hours and an increase in HSP70 expression and a downregulation of the protein levels of client proteins (Src, AKT, Rb and FAK) are shown in western analysis (FIG. 2B). To sum up, Compound 75 is an HSP90/HDAC dual inhibitor that inhibits the activity of HDAC6 and HSP90.

### Example 79 Compound 75 does not cause cytotoxicity to normal cells

The cytotoxicity to normal cells of Compound 75 was evaluated. The results show that Compound 75 does not significantly inhibit the proliferation of human colon normal epithelial cells (CCD841CON) and human lung normal fibroblasts (IMR-90) (IC50>20 µM) (FIGs. 3A and 3B).

### Example 80 Compound 75 reduces IFN-γ-induced PD-L1 and IDO expression by inhibiting STAT1 pathway

Using flow cytometry, we evaluated whether Compound 75 could downregulate IFN-γ-induced PD-L1 performance in different cancer cell lines, namely human colorectal cancer cell lines (HCT116 and LS174T), human pancreatic cancer cell lines (Mia paca-2 and BXPC3), human lung cancer cell line (A549) and mouse colorectal cancer cell line (CT26). It shows that Compound 75 inhibits all IFN-γ-induced cell membrane protein PD-L1 expression (FIG. 4A).

Using western blot, we evaluated whether Compound 75 reduced IFN-γ-induced PD-L1 and IDO performance by inhibiting STAT1 pathway. It was found that Compound 75 downregulated PD-L1 and IDO expression in HCT116 and CT26 cell lines by inhibiting p-STAT1 and STAT1 pathways (FIG. 4B).

### Example 81 Compound 75 inhibits the growth of colorectal cancer tumors in immunocompetent mice

After CT26 was injected subcutaneously into the back of immunocompetent mice (Balb/c), the tumors grew to 50 mm³. Compound 75 was administered intravenously at 10 mg/kg, 25 mg/kg and 50 mg/kg every two days (FIG. 5A). Compound 75 was found to inhibit the growth of colorectal cancer tumors in mice (FIGs. 5B and 5C), with no significant toxicity and no significant change in body weight (FIG. 5D).

### Example 82 Compound 75 does not cause hematotoxicity

Mouse blood cells were assayed to assess whether Compound 75 causes blood toxicity. The results show that white blood cell and lymphocyte counts (FIG. 6A), lymphocyte percentage (FIG. 6B), red blood cell, hemoglobin and platelet counts (FIGs. 7A to 7C) were not significantly different between the treatment and control groups. Taken together, these results suggest that Compound 75 effectively inhibits the growth of murine colorectal cancer and does not cause significant organ toxicity or hematotoxicity in immunocompetent mice. Importantly, while conventional chemotherapeutic agents cause suppression of various immune cells in the body, Compound 75 is not significantly toxic to immune cells, thus maintaining sufficient immune cells in the body to attack tumors.

### Example 83 Compound 75 reduces PD-L1 and IDO expression in tumor regions

Compound 75 was evaluated to determine its ability to reduce PD-L1 and IDO in the tumor microenvironment. The results show that Compound 75 reduces PD-L1 and IDO expressions in the tumor region of mouse colorectal cancer (CT26) with drug concentration dependency (FIGs. 8A and 8B), thereby disrupting the tumor microenvironment.

### Example 84 Compound 75 boosts CD8 cell infiltration in tumor regions and increases granzyme B expression

It is known that TME blocks the infiltration of functional immune cells into the tumor area. The effects of Compound 75 on CD8 cell infiltration in the tumor area were evaluated. From the results, we found that Compound 75 significantly increased CD8 immune cell infiltration in the mouse tumor area (FIG. 9A) with drug concentration dependency. In another aspect, CD8⁺ T cells produce an effector function to kill cancer cells, so we evaluated the ability of CD8⁺ T cells to secrete granzyme B. Therefore, we evaluated the ability of CD8+ T cells to secrete granzyme B. The results show that Compound 75 has a concentration-dependent increase in granzyme B secretion (FIG. 9B).

### Example 85 Compound 75 reduces Treg expression of immune cells in blood

The results of mouse blood analysis show that Compound 75 did not affect the percentage of CD8⁺ cytotoxicity T cells (FIG. 10A) and CD4⁺ helper T cells (FIG. 10B) in blood, but immunosuppressive Treg cell level (FIG. 10C) could be reduced in a concentration dependent manner. We further investigated the reasons for the decrease of Treg in blood. We investigated the serum TGF-β and IL-2 levels in mice treated with Compound 75 and found that Compound 75 significantly reduced serum TGF-β expression in a concentration dependent manner, but had no effect on IL-2 (FIGs. 11A and 11B), suggesting that the reduction in Treg caused by Compound 75 may be due to the reduction in serum TGF-β content.

### Example 86 Compound 75 inhibits tumor growth and enhances the therapeutic effect of Anti-PD1 in mice with colorectal cancer cells

We evaluated the effect of Compound 75 in combination with anti-PD1 (FIG. 12A). The experimental results showed that the treatment effect of Compound 75 (50 mg/kg/2days) and Anti-PD1 (200µg) alone was limited. However, the combined treatment significantly inhibited tumor growth (FIGs. 12B and 12C) and the treatment with Compound 75 was not significantly toxic to mice and there was no significant change in body weight (FIG. 12D). Taken together, Compound 75 enhanced the therapeutic effect of Anti-PD1 on colorectal cancer tumor cells in mice.

### Example 87 Compound 75 combined with chemotherapy enhances antitumor efficacy

We evaluated whether Compound 75 could enhance the tumor suppressive effect of chemotherapeutic agents. We administered control (vehicle), CPT-11 (20 mg/kg), or Compound 75 (50 mg/kg) in combination with CPT-11 to mice and sacrificed them on day 25 as shown in FIG.13A. The results show that Compound 75 significantly enhanced the effect of CPT-11 therapy. In addition, there was no significant difference between the combined therapy and the CPT-11 alone groups in terms of body weight.

### Example 88 Compound 75 increases the percentage of memory T cells and inhibits the recurrence of tumors

We further investigated whether Compound 75 induces the performance of memory T cells to prevent tumor recurrence. We inoculated mouse colorectal cancer cells (CT26) into the back of immunocompetent mice (BALB/c) and gave control or Compound 75 (50 mg/kg) every two days after 3 days, and removed the tumors on day 14. The cancer cells were isolated and cultured *in vitro.* The isolated cancer cells were re-injected into the back of the mice on day 17 and the mice were sacrificed on day 37 (FIG. 14A). The results show that only one mouse suffered cancer recurrence in the group treated with Compound 75 and re-inoculated with control cancer cells (comp.75/control-tumor) and in the group treated with Compound 75 and re-inoculated with Compound 75-treated cancer cells (comp.75/comp.75-tumor). However, in the control group (control/control-tumor), 4 out of 5 cancer recurrences occurred and the tumors were larger (FIGs. 14B, 14C and 14D). This result shows that Compound 75 has the potential to prevent the recurrence of cancer cells.

We further analyzed the central memory T cells (TCM) in the spleen of mice using flow cytometry and showed that the percentage of central memory T cells was significantly increased in the group treated with Compound 75 (FIG. 14E), which effectively inhibited tumor recurrence.

## Claims

1. A compound of formula (I), 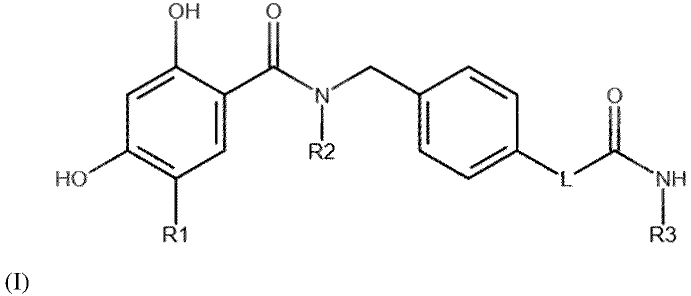
or pharmaceutically acceptable salts, solvates, hydrates, polymorphs, tautomers, stereoisomers, or isotopically enriched derivatives,
wherein:
R₁ is hydrogen or alkyl,
R₂ is 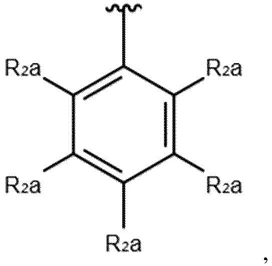 wherein R₂ₐ each independently represents hydrogen, halogen, hydroxy, alkyl, alkoxy, NR'R", heterocyclyl or aryl, or two adjacent R₂ₐ, together with the carbon atoms of the phenyl to which they attached, form a heterocyclyl fused to the phenyl, wherein the heterocyclyl and aryl are unsubstituted or substituted with alkyl, halogen, hydroxy, NR'R", alkoxy or hydroxy, wherein the heterocyclyl contains at least one heteroatom selected from the group consisting of N, O, S and any combination thereof, wherein R' and R" independently represent hydrogen or alkyl;
L is 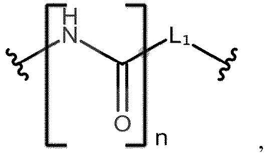 wherein n is 0 or 1, L₁ is a bond, alkylene or alkenylene, with the proviso that when n is 1, L₁ is not a bond, and
R₃ is OH or phenyl optionally substituted with halogen, hydroxy, alkyl, alkoxyor NR'R", wherein R' and R" independently represent hydrogen or alkyl.

2. The compound according to claim 1, wherein R₁ is methyl, ethyl, n-propyl, isopropyl, n-butyl, 2-butyl or 2,2-dimethylethyl.

3. The compound according to claim 1, wherein R₂ is

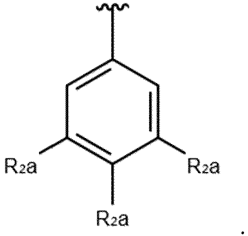

4. The compound according to claim 1, wherein R₂ is phenyl, flourophenyl, chlorophenyl, iodophenyl, benzodioxol, morpholinophenyl, (dimethylamino)phenyl, piperidinylphenyl, methoxyphenyl, or hydroxyphenyl.

5. The compound according to claim 1, wherein L is a bond, or L is 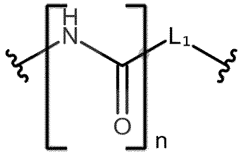 where n is 1 and L₁ is C₁-C₆ alkylene or C₂-C₆ alkenylene.

6. The compound according to claim 1, wherein R₃ is OH or a phenyl substituted with NR'R".

7. The compound according to any of claims 1 to 6, wherein:
(a) R₁ is C₁-C₆ alkyl, R₂ is 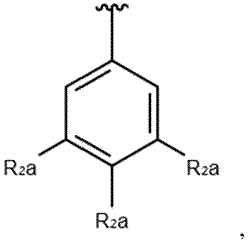 L is a bond, and R₃ is phenyl optionally substituted with halogen, hydroxy, alkyl, alkoxy, or NR'R", wherein R' and R" independently represent hydrogen or alkyl, or
(b) R₁ is C₁-C₆ alkyl, 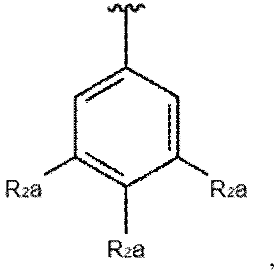 L is a bond or alkenylene and R₃ is OH, or
(c) R₁ is C₁-C₆ alkyl, R₂ is 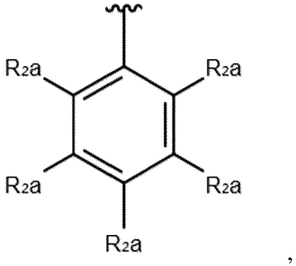 L is 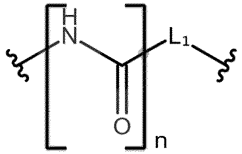 where n is 1 and L₁ is C₁-C₆ alkylene and R₃ is OH, or
(d) R₁ is isopropyl, R₂ is 4-methoxyphenyl, L is 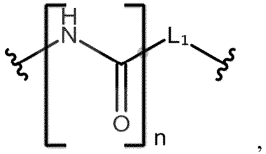 wherein (1) n is 1 and L₁ is alkylene, or (2) n is 0 and L₁ is alkenylene, and R₃ is OH.

8. The compound according to any of claims 1 to 7, which is selected from 4-dihydroxy-N-(4-(hydroxycarbamoyl)benzyl)-5-isopropyl-N-methylbenzamide, N-ethyl-2,4-dihydroxy-N-(4-(hydroxycarbamoyl)benzyl)-5-isopropylbenzamide, 2,4-dihydroxy-N-(4-(hydroxycarbamoyl)benzyl)-5-isopropyl-N-propylbenzamide, 2,4-dihydroxy-N-(4-(hydroxycarbamoyl)benzyl)-5-isopropyl-N-phenylbenzamide, N-(4-fluorophenyl)-2,4-dihydroxy-N-(4-(hydroxycarbamoyl)benzyl)-5-isopropylbenzamide, N-(4-chlorophenyl)-2,4-dihydroxy-N-(4-(hydroxycarbamoyl)benzyl)-5-isopropylbenzamide, 2,4-dihydroxy-N-(4-(hydroxycarbamoyl)benzyl)-N-(4-iodophenyl)-5-isopropylbenzamide, N-(benzo[d][1,3]dioxol-5-yl)-2,4-dihydroxy-N-(4-(hydroxycarbamoyl)benzyl)-5-isopropylbenzamide, 2,4-dihydroxy-N-(4-(hydroxycarbamoyl)benzyl)-5-isopropyl-N-(4-morpholinophenyl)benzamide, N-(4-(dimethylamino)phenyl)-2,4-dihydroxy-N-(4-(hydroxycarbamoyl)benzyl)-5-isopropylbenzamide, 2,4-dihydroxy-N-(4-(hydroxycarbamoyl)benzyl)-5-isopropyl-N-(4-(4-methylpiperazin-1-yl)phenyl)benzamide, 2,4-dihydroxy-N-(4-(hydroxycarbamoyl)benzyl)-5-isopropyl-N-(4-(piperidin-1-yl)phenyl)benzamide, 2,4-dihydroxy-N-(4-hydroxycarbamoyl-benzyl)-5-isopropyl-N-(4-methoxy-phenyl)-benzamide, and 2,4-dihydroxy-N-(4-hydroxycarbamoyl-benzyl)-N-(4-hydroxyphenyl)-5-isopropyl-benzamide.

9. A pharmaceutical composition comprising a therapeutically effective amount of the compound according to any of claims 1 to 8 and optionally pharmaceutically acceptable excipients.

10. The pharmaceutical composition according to claim 9, for use in a method for amelioration and/or treatment of tumors, inhibiting tumor growth, and/or inhibiting tumor recurrence.

11. The pharmaceutical composition for the use according to claim 10, wherein the method further comprises administering an immune checkpoint inhibitor, a tumor-targeting inhibitor, or a chemotherapy drug.

12. The pharmaceutical composition for the use according to claim 11, wherein:
the immune checkpoint is PD-1,
the tumor-targeting inhibitor is an antibody,
the chemotherapy drug is an alkylating agent, antimetabolite, anthracycline, topoisomerase I and II inhibitor, mitotic inhibitor, platinum based drug, steroid or anti-angiogenic agent.

13. The pharmaceutical composition for the use according to claim 10, wherein the tumor is a solid tumor.

14. The pharmaceutical composition for the use according to claim 10, wherein the tumor is selected from colorectal cancer, pancreatic carcinoma, small cell lung cancer, non-small cell lung cancer, renal cell carcinoma, breast cancer, head and neck cancer, prostate cancer, malignant gliomas, osteosarcoma, gastric cancer, malignant mesothelioma, multiple myeloma, ovarian cancer, synovial sarcoma, thyroid cancer, or melanoma.

15. The pharmaceutical composition according to claim 9, for use in a method for removing immune suppression from tumor microenvironments or stimulating an immune system against tumors, inhibiting histone deacetylase 6 (HDAC6) in tumor microenvironments, inducing infiltration of cytotoxic T cells in tumor microenvironments, inhibiting heat shock protein 90 (HSP90) in tumor microenvironments, reducing Treg cell level, blocking signal transducer and activator of transcription 1 (STAT1) pathway induced by interferon-gamma (IFN-γ), lowering programmed death ligand 1 (PD-L1) or indoleamine-pyrrole 2,3-dioxygenase (IDO) expression of the tumor, inhibiting acetylation of α-tubulin and histone, inducing granzyme B expression, destabilizing proteins for tumor growth, increasing heat shock protein 70 (HSP70) expression, and/or reducing expression of Src, AKT, retinoblastoma protein (Rb) or focal adhesion kinase (FAK).

## Patentansprüche

1. Verbindung von Formel (I): 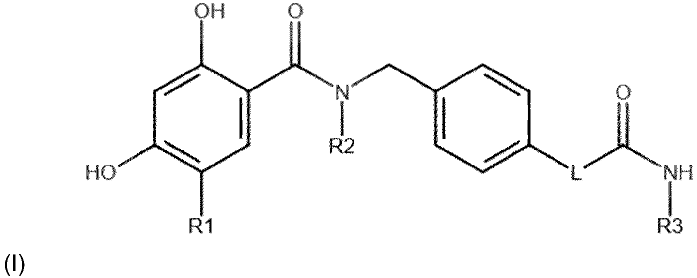
oder pharmazeutisch annehmbare Salze, Solvate, Hydrate, Polymorphe, Tautomere, Stereoisomere oder isotopisch angereicherte Derivate,
wobei:
R₁ Wasserstoff oder Alkyl ist,
R₂ 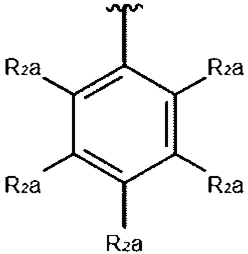 ist, wobei R₂ₐ jeweils unabhängig Wasserstoff, Halogen, Hydroxy, Alkyl, Alkoxy, NR'R", Heterocyclyl oder Aryl darstellt, oder zwei benachbarte R₂ₐ zusammen mit den Kohlenstoffatomen des Phenyls, an das sie angelagert sind, ein an das Phenyl kondensiertes Heterocyclyl bilden, wobei das Heterocyclyl und Aryl unsubstituiert oder mit Alkyl, Halogen, Hydroxy, NR'R", Alkoxy oder Hydroxy substituiert sind, wobei der Heterocyclyl mindestens ein Heteroatom enthält, das ausgewählt ist aus der Gruppe, bestehend aus N, O, S oder einer Kombination davon, wobei R' und R" unabhängig Wasserstoff oder Alkyl darstellen;
L 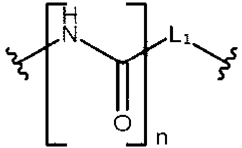 ist, wobei n 0 oder 1 ist, L₁ eine Bindung, eine Alkylen- oder Alkenylengruppe ist, mit der Maßgabe, dass, wenn n 1 ist, L₁ keine Bindung ist, und
R₃ OH oder Phenyl ist, optional substituiert mit Halogen, Hydroxy, Alkyl, Alkoxy oder NR'R" wobei R' und R" unabhängig Wasserstoff oder Alkyl darstellen.

2. Verbindung nach Anspruch 1, wobei R₁ Methyl, Ethyl, n-Propyl, Isopropyl, n-Butyl, 2-Butyl oder 2,2-Dimethylethyl ist.

3. Verbindung nach Anspruch 1, wobei R₂ 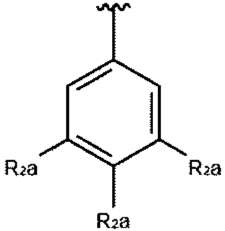 ist.

4. Verbindung nach Anspruch 1, wobei R₂ Phenyl, Fluorphenyl, Chlorphenyl, lodphenyl, Benzodioxol, Morpholinophenyl, (Dimethylamino)phenyl, Piperidinylphenyl, Methoxyphenyl oder Hydroxyphenyl ist.

5. Verbindung nach Anspruch 1, wobei L eine Bindung ist oder L 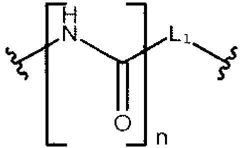 ist, wobei n 1 ist und L₁ C₁-C₆-Alkylen oder C₂-C₆-Alkenylen ist.

6. Verbindung nach Anspruch 1, wobei R₃ OH oder ein mit NR'R" substituiertes Phenyl ist.

7. Verbindung nach einem der Ansprüche 1 bis 6, wobei:
(a) R₁ C₁-C₆-Alkyl ist, R₂ 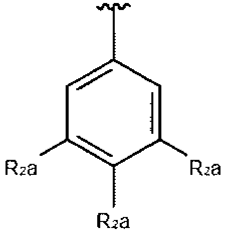 ist, L **eine** Bindung ist, und R₃ Phenyl ist, optional substituiert mit Halogen, Hydroxy, Alkyl, Alkoxy oder NR'R", wobei R' und R" unabhängig Wasserstoff oder Alkyl darstellen, oder
(b) R₁ C₁-C₆-Alkyl ist, 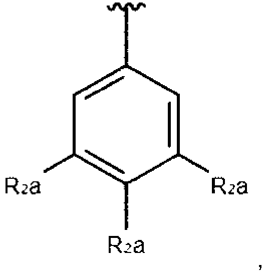 L eine Bindung oder Alkenylen ist und R₃ OH ist, oder
(c) R₁ C₁-C₆ Alkyl ist, R₂ 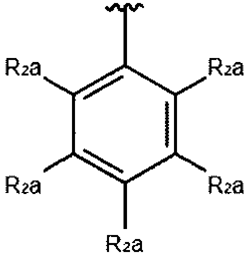 ist, L 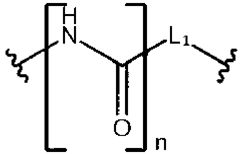 ist, wobei n 1 ist und L₁ C₁-C₆-Alkylen ist und R₃ OH ist, oder
(d) R₁ Isopropyl ist, R₂ 4-Methoxyphenyl ist, L 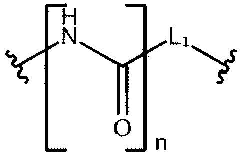 ist, wobei (1) n gleich 1 ist und L₁ Alkylen ist, oder (2) n gleich 0 ist und L₁ Alkenylen ist, und R₃ OH ist.

8. Verbindung nach einem der Ansprüche 1 bis 7, die ausgewählt ist aus 4-Dihydroxy-N-(4-(hydroxycarbamoyl)benzyl)-5-isopropyl-N-methylbenzamid, N-Ethyl-2,4-dihydroxy-N-(4-(hydroxycarbamoyl)benzyl)-5-isopropylbenzamid, 2,4-Dihydroxy-N-(4-(hydroxycarbamoyl)benzyl)-5-isopropyl-N-propylbenzamid, 2,4-Dihydroxy-N-(4-(hydroxycarbamoyl)benzyl)-5-isopropyl-N-phenylbenzamid, N-(4-Fluorphenyl)-2,4-dihydroxy-N-(4-(hydroxycarbamoyl)benzyl)-5-isopropylbenzamid, N-(4-Chlorphenyl)-2,4-dihydroxy-N-(4-(hydroxycarbamoyl)benzyl)-5-isopropylbenzamid, 2,4-Dihydroxy-N-(4-(hydroxycarbamoyl)benzyl)-N-(4-iodphenyl)-5-isopropylbenzamid, N-(Benzo[d][1,3]dioxol-5-yl)-2,4-dihydroxy-N-(4-(hydroxycarbamoyl)benzyl)-5-isopropylbenzamid, 2,4-Dihydroxy-N-(4-(hydroxycarbamoyl)benzyl)-5-isopropyl-N-(4-morpholinophenyl)benzamid, N-(4-(Dimethylamino)phenyl)-2,4-dihydroxy-N-(4-(hydroxycarbamoyl)benzyl)-5-isopropylbenzamid, 2,4-Dihydroxy-N-(4-(hydroxycarbamoyl)benzyl)-5-isopropyl-N-(4-(4-methylpiperazin-1-yl)phenyl)benzamid, 2,4-Dihydroxy-N-(4-(hydroxycarbamoyl)benzyl)-5-isopropyl-N-(4-(piperidin-1-yl)phenyl)benzamid, 2,4-Dihydroxy-N-(4-hydroxycarbamoylbenzyl)-5-isopropyl-N-(4-methoxyphenyl)-benzamid und 2,4-Dihydroxy-N-(4-hydroxycarbamoylbenzyl)-N-(4-hydroxyphenyl)-5-isopropylbenzamid.

9. Pharmazeutische Zusammensetzung, umfassend eine therapeutisch wirksame Menge der Verbindung nach einem der Ansprüche 1 bis 8 und optional pharmazeutisch annehmbare Hilfsstoffe.

10. Pharmazeutische Zusammensetzung nach Anspruch 9 zur Verwendung in einem Verfahren zur Linderung und/oder Behandlung von Tumoren, zur Hemmung des Tumorwachstums und/oder zur Hemmung eines Tumorrezidivs.

11. Pharmazeutische Zusammensetzung zur Verwendung nach Anspruch 10, wobei das Verfahren ferner ein Verabreichen eines Immun-Checkpoint-Hemmers, eines tumorspezifischen Hemmers oder eines Chemotherapeutikums umfasst.

12. Pharmazeutische Zusammensetzung zur Verwendung nach Anspruch 11, wobei:
der Immun-Checkpoint PD-1 ist,
der tumorspezifische Hemmer ein Antikörper ist,
das Chemotherapeutikum ein Alkylierungsmittel, ein Antimetabolit, ein Anthrazyklin, ein Topoisomerase-I- und -II-Hemmer, ein Mitosehemmer, ein platinbasiertes Medikament, ein Steroid oder ein antiangiogenes Mittel ist.

13. Pharmazeutische Zusammensetzung zur Verwendung nach Anspruch 10, wobei der Tumor ein solider Tumor ist.

14. Pharmazeutische Zusammensetzung zur Verwendung nach Anspruch 10, wobei der Tumor ausgewählt ist aus kolorektalem Karzinom, Pankreaskarzinom, kleinzelligem Lungenkarzinom, nicht-kleinzelligem Lungenkarzinom, Nierenzellkarzinom, Brustkrebs, Kopf-Hals-Krebs, Prostatakrebs, malignen Gliomen, Osteosarkom, Magenkrebs, malignem Mesotheliom, multiplem Myelom, Eierstockkrebs, Synovialsarkom, Schilddrüsenkrebs oder Melanom.

15. Pharmazeutische Zusammensetzung nach Anspruch 9 zur Verwendung in einem Verfahren zur Aufhebung der Immunsuppression in Tumormikroumgebungen oder zur Stimulierung des Immunsystems gegen Tumore, zum Hemmen von Histon-Deacetylase 6 (HDAC6) in Tumormikroumgebungen, zur Induktion der Infiltration zytotoxischer T-Zellen in Tumormikroumgebungen, zum Hemmen des Hitzeschockproteins 90 (HSP90) in Tumormikroumgebungen, zum Verringern des Treg-Zellspiegels, zum Blockieren des durch Interferon-gamma (IFN-γ) induzierten Signaltransduktors und Transkriptionsaktivators 1 (STAT1)-Signalweg, zum Senken der Expression des programmierten Zelltod-Liganden 1 (PD-L1) oder der Indolamin-Pyrrol-2,3-Dioxygenase (IDO) des Tumors, zum Hemmen einer Acetylierung von α-Tubulin und Histon, zur Induktion der Granzym-B-Expression, zum Destabilisieren von Proteinen, die das Tumorwachstum fördern, zum Erhöhen der Expression des Hitzeschockproteins 70 (HSP70) und/oder zum Verringern der Expression von Src, AKT, Retinoblastom-Protein (Rb) oder der fokalen Adhäsionskinase (FAK).

## Revendications

1. Composé de formule (I), 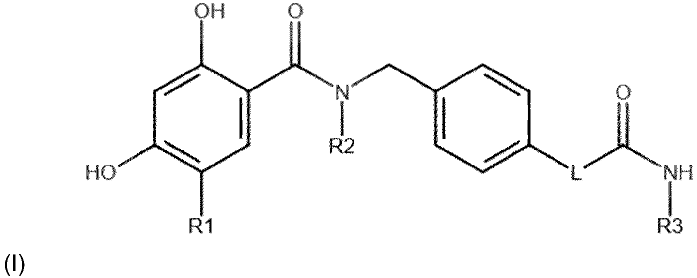
ou leurs sels, solvates, hydrates, polymorphes, tautomères, stéréoisomères ou dérivés enrichis isotopiquement pharmaceutiquement acceptables,
dans lequel :
R₁ est un hydrogène ou un alkyle,
R₂ est 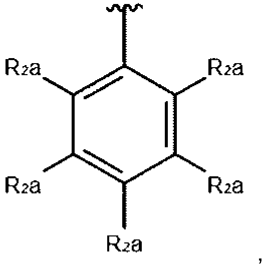 dans lequel R₂ₐ représente chacun indépendamment un hydrogène, un halogène, un groupe hydroxy, un alkyle, un groupe alcoxy, NR'R", un hétérocyclyle ou un aryle, ou deux R2a adjacents, conjointement avec les atomes de carbone du phényle auquel ils sont attachés, forment un hétérocyclyle fusionné au phényle, dans lequel l'hétérocyclyle et l'aryle sont non substitués ou substitués par un alkyle, un halogène, un groupe hydroxy, un NR'R", un groupe alcoxy ou un groupe hydroxy, dans lequel l'hétérocyclyle contient au moins un hétéroatome choisi dans le groupe constitué par N, O, S et une quelconque combinaison de ceux-ci, dans lequel R' et R" représentent indépendamment un hydrogène ou un alkyle ;
L est 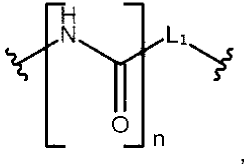 dans lequel n est 0 ou 1, L₁ est une liaison, un alkylène ou un alcénylène, à condition que lorsque n est 1, L₁ ne soit pas une liaison, et
R₃ est OH ou un phényle facultativement substitué par un halogène, un groupe hydroxy, un alkyle, un groupe alcoxy ou NR'R", dans lequel R' et R" représentent indépendamment un hydrogène ou un alkyle.

2. Composé selon la revendication 1, dans lequel R₁ est le méthyle, l'éthyle, le n-propyle, l'isopropyle, le n-butyle, le 2-butyle ou le 2,2-diméthyléthyle.

3. Composé selon la revendication 1, dans lequel R₂ est

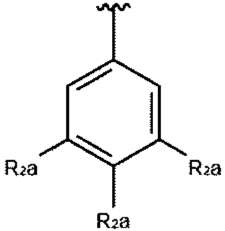

4. Composé selon la revendication 1, dans lequel R₂ est le phényle, le fluorophényle, le chlorophényle, l'iodophényle, le benzodioxol, le morpholinophényle, le (diméthylamino)phényle, le pipéridinylphényle, le méthoxyphényle ou l'hydroxyphényle.

5. Composé selon la revendication 1, dans lequel L est une liaison, ou L est 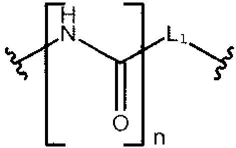 où n est 1 et L₁ est un alkylène en C₁-C₆ ou un alcénylène en C₂-C₆.

6. Composé selon la revendication 1, dans lequel R₃ est OH ou un phényle substitué par NR'R".

7. Composé selon l'une quelconque des revendications 1 à 6, dans lequel :
(a) R₁ est un alkyle en C₁-C₆, R₂ est 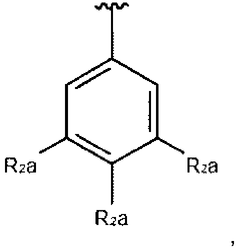 L est une liaison, et R₃ est un phényle facultativement substitué par un halogène, un groupe hydroxy, un alkyle, un groupe alcoxy ou NR'R", dans lequel R' et R" représentent indépendamment un hydrogène ou un alkyle, ou
(b) R₁ est un alkyle en C₁-C₆, 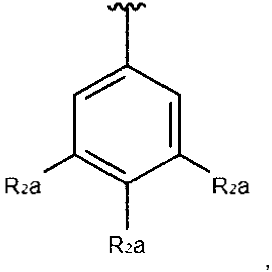 L est une liaison ou un alcénylène et R₃ est OH, ou
(c) R₁ est un alkyle en C₁-C₆, R₂ est 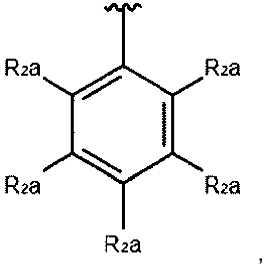 L est 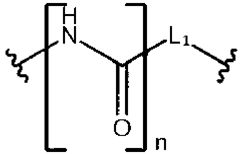 où n est 1 et L₁ est un alkylène en C₁-C₆ et R₃ est OH, ou
(d) R₁ est l'isopropyle, R₂ est le 4-méthoxyphényle, L est 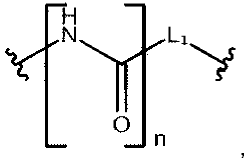 dans lequel (1) n est 1 et L₁ est un alkylène, ou (2) n est 0 et L₁ est un alcénylène, et R₃ est OH.

8. Composé selon l'une quelconque des revendications 1 à 7, qui est choisi parmi le 4-dihydroxy-N-(4-(hydroxycarbamoyl)benzyl)-5-isopropyl-N-méthylbenzamide, le N-éthyl-2,4- dihydroxy-N-(4-(hydroxycarbamoyl)benzyl)-5-isopropylbenzamide, le 2,4-dihydroxy- N-(4-(hydroxycarbamoyl)benzyl)-5-isopropyl-N-propylbenzamide, le 2,4-dihydroxy-N- (4-(hydroxycarbamoyl)benzyl)-5-isopropyl-N-phénylbenzamide, le N-(4-fluorophényl)- 2,4-dihydroxy-N-(4-(hydroxycarbamoyl)benzyl)-5-isopropylbenzamide, le N-(4-chlorophényl)-2,4-dihydroxy-N-(4-(hydroxycarbamoyl)benzyl)-5- isopropylbenzamide, le 2,4-dihydroxy-N-(4-(hydroxycarbamoyl)benzyl)-N-(4-iodophényl)-5-isopropylbenzamide, le N-(benzo[d][1,3]dioxol-5-yl)-2,4-dihydroxy-N- (4-(hydroxycarbamoyl)benzyl)-5-isopropylbenzamide, le 2,4-dihydroxy-N-(4- (hydroxycarbamoyl)benzyl)-5-isopropyl-N-(4-morpholinophényl)benzamide, le N-(4- (diméthylamino)phényl)-2,4-dihydroxy-N-(4-(hydroxycarbamoyl)benzyl)-5- isopropylbenzamide, le 2,4-dihydroxy-N-(4-(hydroxycarbamoyl)benzyl)-5-isopropyl-N- (4-(4-méthylpipérazin-1-yl)phényl)benzamide, le 2,4-dihydroxy-N-(4-(hydroxycarbamoyl)benzyl)-5-isopropyl-N-(4-(pipéridin-1-yl)phényl)benzamide, le 2,4- dihydroxy-N-(4-hydroxycarbamoyl-benzyl)-5-isopropyl-N-(4-méthoxy-phényl)- benzamide, et le 2,4-dihydroxy-N-(4-hydroxycarbamoyl-benzyl)-N-(4-hydroxy- phényl)-5-isopropylbenzamide.

9. Composition pharmaceutique comprenant une quantité thérapeutiquement efficace du composé selon l'une quelconque des revendications 1 à 8 et, facultativement, des excipients pharmaceutiquement acceptables.

10. Composition pharmaceutique selon la revendication 9, destinée à être utilisée dans un procédé d'amélioration et/ou de traitement de tumeurs, d'inhibition de la croissance tumorale et/ou d'inhibition de la récidive tumorale.

11. Composition pharmaceutique pour l'utilisation selon la revendication 10, dans laquelle le procédé comprend en outre l'administration d'un inhibiteur de point de contrôle immunitaire, d'un inhibiteur ciblant la tumeur ou d'un médicament de chimiothérapie.

12. Composition pharmaceutique pour l'utilisation selon la revendication 11, dans laquelle :
le point de contrôle immunitaire est le PD-1,
l'inhibiteur ciblant la tumeur est un anticorps,
le médicament de chimiothérapie est un agent alkylant, un antimétabolite, une anthracycline, un inhibiteur de la topo-isomérase I et II, un inhibiteur mitotique, un médicament à base de platine, un stéroïde ou un agent anti-angiogénique.

13. Composition pharmaceutique pour l'utilisation selon la revendication 10, dans laquelle la tumeur est une tumeur solide.

14. Composition pharmaceutique pour l'utilisation selon la revendication 10, dans laquelle la tumeur est choisie parmi le cancer colorectal, le carcinome du pancréas, le cancer du poumon à petites cellules, le cancer du poumon non à petites cellules, le carcinome rénal à cellules, le cancer du sein, le cancer de la tête et du cou, le cancer de la prostate, les gliomes malins, l'ostéosarcome, le cancer de l'estomac, le mésothéliome malin, le myélome multiple, le cancer des ovaires, le sarcome synovial, le cancer de la thyroïde ou le mélanome.

15. Composition pharmaceutique selon la revendication 9, destinée à être utilisée dans un procédé d'élimination de l'immunosuppression des microenvironnements tumoraux ou de stimulation d'un système immunitaire contre les tumeurs, d'inhibition de l'histone désacétylase 6 (HDAC6) dans les microenvironnements tumoraux, d'induction de l'infiltration de lymphocytes T cytotoxiques dans les microenvironnements tumoraux, d'inhibition de la protéine de choc thermique 90 (HSP90) dans les microenvironnements tumoraux, de réduction du niveau de lymphocytes T régulateurs Treg, de blocage de la voie du transducteur de signal et activateur de transcription 1 (STAT1) induite par l'interféron-gamma (IFN-γ), de diminution du ligand 1 de la mort programmée (PD-L1) ou de l'indoléamine-pyrrole 2,3-dioxygénase (IDO) de la tumeur, d'inhibition de l'acétylation de l'α-tubuline et de l'histone, d'induction de l'expression du granzyme B, de déstabilisation des protéines pour la croissance tumorale, d'augmentation de l'expression de la protéine de choc thermique 70 (HSP70), et/ou de réduction de l'expression de Src, AKT, de la protéine du rétinoblastome (Rb) ou de la kinase d'adhérence focale (FAK).
